# EUROPÄISCHE PATENTSCHRIFT

(11) **EP 0 646 473 B1**
(45) Veröffentlichungstag und Bekanntmachung des Hinweises auf die Patenterteilung: **29.12.1997**
(21) Anmeldenummer: 94810542.4
(22) Anmeldetag: 21.09.1994
(51) Int. Cl.: B41M 5/15, B41M 5/145, B41M 5/165, B41M 5/30, C07D 493/10

(54) **Farbbildnergemisch**
Mixture of colour formers
Mélange de formateurs de couleur

(30) Priorität: 30.09.1993 CH 2946/93
(43) Veröffentlichungstag der Anmeldung: 05.04.1995
(73) Patentinhaber: Ciba Specialty Chemicals Holding Inc., 4057 Basel (CH)
(72) Erfinder: Zink, Rudolf, CH-4106 Therwil (CH); Huber, Klaus Dr., D-79110 Freiburg (DE)

(56) Entgegenhaltungen:
- EP-A- 0 193 200
- EP-A- 0 201 225
- EP-A- 0 561 738
- US-A- 5 071 480

## Beschreibung

Die vorliegende Erfindung betrifft ein Farbbildnergemisch und seine Verwendung in druckempfindlichen und insbesondere wärmeempfindlichen Aufzeichnungsmaterialien sowie neue Farbildner und die Herstellung dieser neuen Farbbildner.

Farbbildnergemische, die in druck- und wärmeempfindlichen Aufzeichnungssystemen farbstarke und stabile Färbungen aufweisen, sind z.B. aus der US-A-5,071,480 bekannt.

Die Aufgabe der vorliegenden Erfindung besteht darin, ein Farbbildnergemisch, insbesondere für die Thermographie bereitzustellen, das Farbbildnergemische aus dem Stand der Technik hinsichtlich Lagerstabilität und Papierweisse (Hintergrund) noch übertrifft.

Gegenstand der vorliegenden Erfindung ist daher ein Farbbildnergemisch, enthaltend
(a) eine Verbindung der Formel 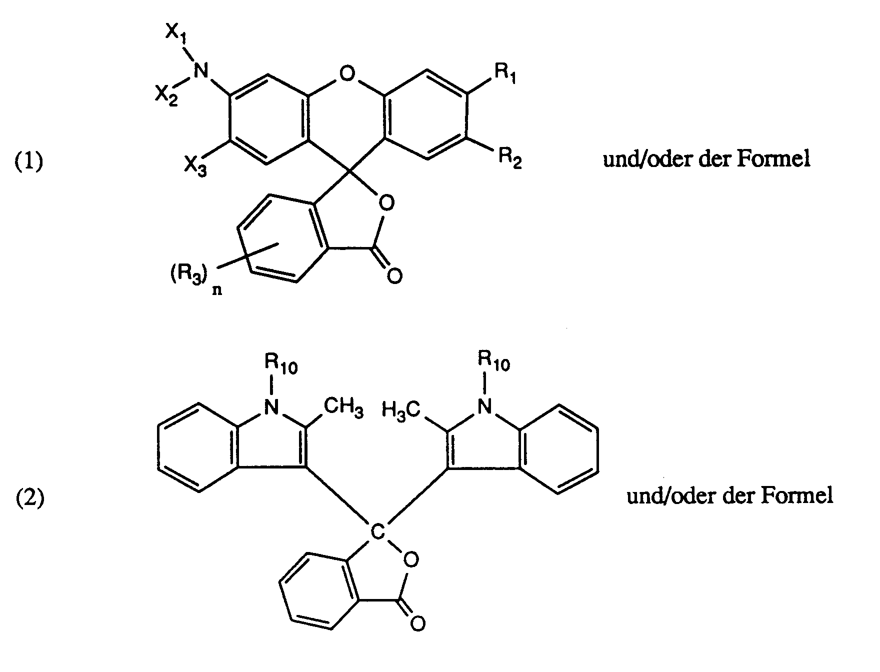 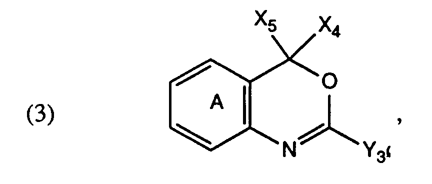 und
(b) eine Verbindung der Formel 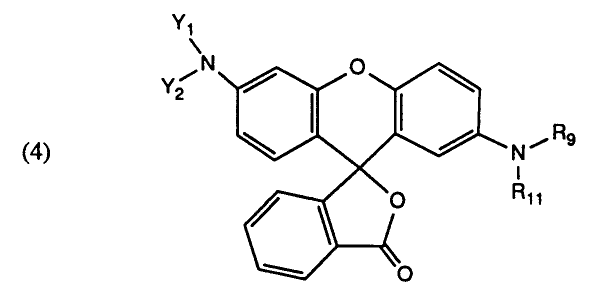 oder ein Farbbildnergemisch, enthaltend mindestens zwei Verbindungen der Komponente (b),
   worin
   - R₁: Wasserstoff, Hydroxy, Halogen oder C₁-C₄-Alkyl;
   - R₂: Wasserstoff; Nitro; SO₂R₄; SO₂OR₅; SO₂NR₆R₇; COR₈; CONR₆R₇; oder C₁-C₄-Halogenalkyl; nicht substituierter oder durch Halogen oder Hydroxy substituierter 2-Triazinyl- oder 1-Benztriazolylrest;
   - R₃: Halogen; Nitro; C₁-C₄-Alkyl; C₁-C₄-Halogenalkyl; Amino; mono-C₁-C₄-Alkylamino; di-C₁-C₄-Alkylamino; oder COR₈;
   - R₄: C₁-C₈-Alkyl; C₁-C₈-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₄-Alkyl;
   - R₅: Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
   - R₆ und R₇: unabhängig voneinander Wasserstoff; oder C₁-C₈-Alkyl; oder
   - R₆ und R₇: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
   - R₈: Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
   - R₉ und R₁₀: unabhängig voneinander C₁-C₈-Alkyl; oder 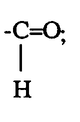
   - R₁₁: Wasserstoff oder C₁-C₅-Alkyl;
   - R₁₂ und R₁₃,: unabhängig voneinander, je Wasserstoff, unsubstituiertes oder durch Halogen, Hydroxy, Cyano oder C₁-C₅-Alkoxy substituiertes Alkyl mit höchstens 12 Kohlenstoffatomen, C₅-C₇-Cycloalkyl oder unsubstituiertes oder durch Halogen, Cyano, C₁-C₅-Alkyl oder C₁-C₅-Alkoxy ringsubstituiertes Phenylalkyl oder Phenyl oder
   - R₁₂ und R₁₃: zusammen mit dem sie verbindenden Stickstoffatom einen fünf- oder sechsgliedrigen heterocyclischen Rest;
   - X₁ und X₂,: unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄-Alkyl; 2-Tetrahydrofuranyl, oder
   - X₁ und X₂: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
   - X₃: Wasserstoff oder C₁-C₄-Alkyl;
   - X₄ und X₅: unabhängig voneinander unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl oder Phenyl-C₁-C₄-Alkyl; einen 2-Pyrrolyl-, 2-Thienyl-, 2- oder 3-Indolyl-, 2-Benzofuranyl- oder 2-Naphthothienylrest;
   - Y₁ und Y₂: unabhängig voneinander C₁-C₅-Alkyl;
   - Y₃: Wasserstoff; C₁-C₅-Alkyl; C₅-C₇-Cycloalkyl, unsubstituiertes oder im Phenylrest durch Halogen, C₁-C₄-Alkyl, C₁-C₄-Halogenalkyl, C₁-C₄-Alkoxy substituiertes Phenyl;
   - der Benzolring A: durch Halogen; Cyano; Nitro; C₁-C₅-Alkyl; C₁-C₅-Alkoxy; C₁-C₅-Alkylthio; C₁-C₅-Alkylcarbonyl; C₁-C₅-Alkoxycarbonyl; oder NR₁₂R₁₃ substituiert sein kann; und
   - n: 0; 1; 2; 3; oder 4;
bedeuten.

Die Komponenten der Formeln (1), (2), (3) und (4) können als Einzelverbindungen oder als Gemische vorhanden sein.

In der Literatur werden die einzelnen Substituentenposititionen im Fluoranring unterschiedlich numeriert. In der vorliegenden Anmeldung wird die folgende Numerierung verwendet:

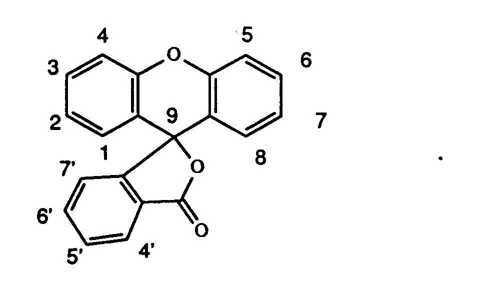

Im Rahmen der vorstehenden Definition haben die jeweiligen Substituenten insbesondere die folgenden Bedeutungen:

Halogen ist Fluor, Chlor oder Brom, insbesondere Fluor oder Chlor.

Alkyl bedeutet im Rahmen der jeweiligen Definition geradkettiges oder verzweigtes Alkyl. Beispiele solcher Alkylreste sind Methyl, Ethyl, n-Propyl, Isopropyl, n-Butyl, 1-Methylbutyl, sek.Butyl, tert.Butyl, n-Pentyl, Amyl, Isoamyl, n-Hexyl, 2-Ethyl-hexyl, n-Heptyl, n-Octyl, Isooctyl, 1,1,3,3-Tetramethylbutyl.

Beispiele für Halogenalkyl sind insbesondere die C₁-C₂-Halogenalkylreste, wie Trichlormethyl, Trifluormethyl, Dichlorfluormethyl, Difluorchlormethyl, Perchlorethyl, 1,1,2,2-Tetrachlorethyl, 1,1,2,2-Tetrafluorethyl, 2,2,2-Trichlorethyl. R₅ als C₁-C₈-Halogenalkyl sind insbesondere die zuvor genannten Halogenalkyle, aber auch Alkylradikale, bei denen alle oder zumindest der überwiegende Teil der C-H Bindungen durch C-Cl oder C-F ersetzt ist.

Alkoxy ist insbesondere Methoxy, Ethoxy, n-Propoxy, Isopropoxy, n-Butoxy, Isobutoxy, sek.Butoxy und tert.Butoxy, C₁-C₄Alkoxy-C₁-C₄Alkyl insbesondere Methoxymethyl, Methoxyethyl, Ethoxymethyl oder Ethoxyethyl.

Mono-C₁-C₅-Alkylamino ist insbesondere Methylamino, Ethylamino, Propylamino, Butylamino und Pentylamino. Di-C₁-C₅-Alkylamino umfaßt sowohl die gemischten wie auch die gleichnamig substituierten Radikale, wie Methylethylamino, Dimethylamino, Diethylamino, Methylpropylamino, Methylbutylamino, Di-n-propylamino, Di-isopropylamino, Di-n-butylamino und Di-n-pentylamino etc..

In Phenyl-C₁-C₄-Alkyl und Phenyl-C₁-C₄-Alkoxy kann der Phenylrest über eine geradkettige oder verzweigte Alkyl- bzw. Alkoxykette gebunden sein. Bevorzugt ist Phenethyl, Benzyl und Phenylmethoxy.

Der Phenylrest in Pheny-C₁-C₄-Alkyl, Pheny-C₁-C₄-Alkoxy bzw. Phenyl ist vorzugweise unsubstituiert oder bis zu dreifach gleich oder verschieden durch die genannten Substituenten substituiert.

Beispiele für C₃-C₅-Alkenyl sind Allyl, 1-Propenyl oder 2-Pentenyl, Isopropenyl oder 2-Butenyl. Allyl ist bevorzugt. C₄-C₇-Cyclohexyl bedeutet Cyclobutyl, Cyclopentyl, Cyclohexyl oder Cycloheptyl, wobei Cyclohexyl im Vordergrund des Interesses steht.

Im Vordergrund des Interesses steht ein Farbbildnergemisch, enthaltend
(a) eine Verbindung der Formel 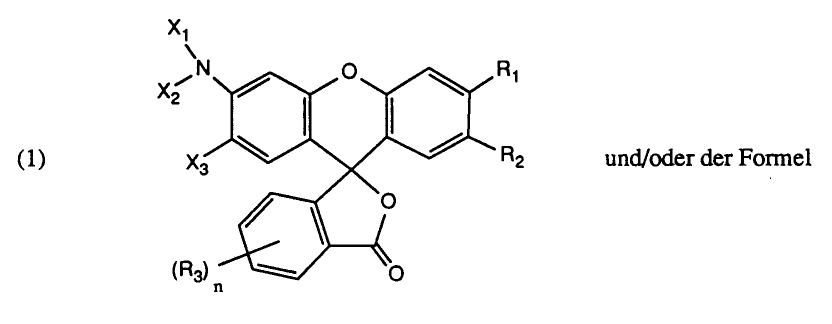 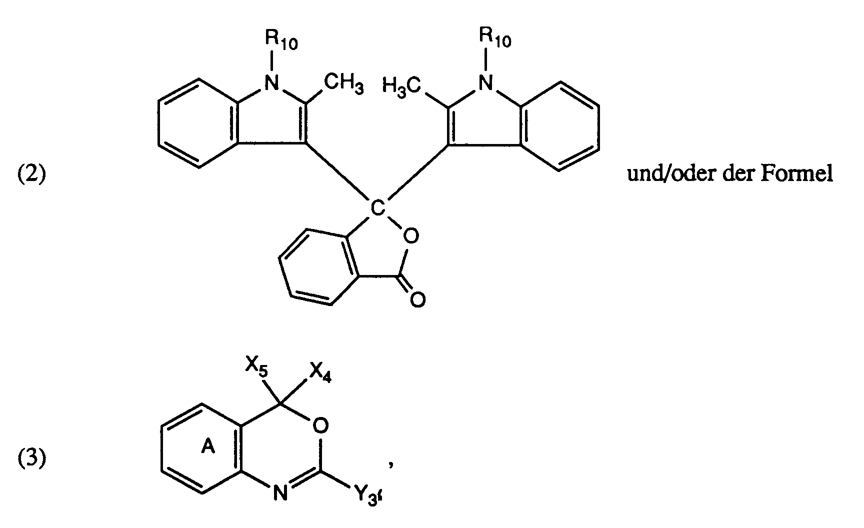 und
(b) eine Verbindung der Formel 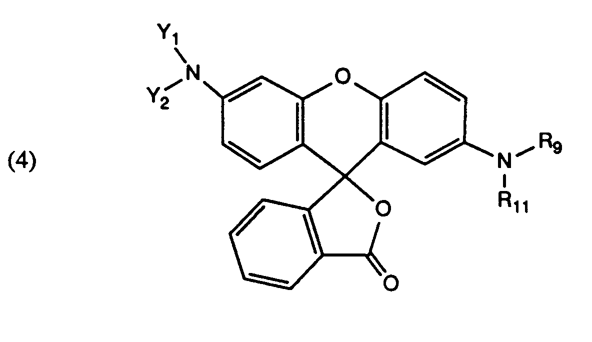 worin
   - R₁: Wasserstoff, Hydroxy, Halogen oder C₁-C₄-Alkyl;
   - R₂: Wasserstoff; Nitro; SO₂R₄; SO₂OR₅; SO₂NR₆R₇; COR₈; CONR₆R₇; oder C₁-C₄-Halogenalkyl; nicht substituierter oder durch Halogen oder Hydroxy substituierter 2-Triazinyl- oder 1-Benztriazolylrest;
   - R₃: Halogen; Nitro; C₁-C₄-Alkyl; C₁-C₄-Halogenalkyl; Amino; mono-C₁-C₄-Alkylamino; di-C₁-C₄-Alkylamino; oder COR₈;
   - R₄: C₁-C₈-Alkyl; C₁-C₈-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₄-Alkyl;
   - R₅: Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
   - R₆ und R₇: unabhängig voneinander Wasserstoff; oder C₁-C₈-Alkyl; oder
   - R₆ und R₇: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
   - R₈: Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
   - R₉ und R₁₀: unabhängig voneinander C₁-C₈-Alkyl; oder 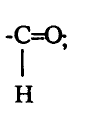
   - R₁₁: Wasserstoff oder C₁-C₅-Alkyl;
   - R₁₂ und R₁₃,: unabhängig voneinander, je Wasserstoff, unsubstituiertes oder durch Halogen, Hydroxy, Cyano oder C₁-C₅-Alkoxy substituiertes Alkyl mit höchstens 12 Kohlenstoffatomen, C₅-C₇-Cycloalkyl oder unsubstituiertes oder durch Halogen, Cyano, C₁-C₅-Alkyl oder C₁-C₅-Alkoxy ringsubstituiertes Phenylalkyl oder Phenyl oder
   - R₁₂ und R₁₃: zusammen mit dem sie verbindenden Stickstoffatom einen fünf- oder sechsgliedrigen heterocyclischen Rest;
   - X₁ und X₂,: unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄-Alkyl; 2-Tetrahydrofuranyl, oder
   - X₁ und X₂: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
   - X₃: Wasserstoff oder C₁-C₄-Alkyl;
   - X₄ und X₅: unabhängig voneinander unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl oder Phenyl-C₁-C₄-Alkyl; einen 2-Pyrrolyl-, 2-Thienyl-, 2- oder 3-Indolyl-, 2-Benzofuranyl- oder 2-Naphthothienylrest;
   - Y₁ und Y₂: unabhängig voneinander C₁-C₅-Alkyl;
   - Y₃: Wasserstoff; C₁-C₅-Alkyl; C₅-C₇-Cycloalkyl, unsubstituiertes oder im Phenylrest durch Halogen, C₁-C₄-Alkyl, C₁-C₄-Halogenalkyl, C₁-C₄-Alkoxy substituiertes Phenyl;
   - der Benzolring A: durch Halogen; Cyano; Nitro; C₁-C₅-Alkyl; C₁-C₅-Alkoxy; C₁-C₅-Alkylthio; C₁-C₅-Alkylcarbonyl; C₁-C₅-Alkoxycarbonyl; oder NR₁₂R₁₃ substituiert sein kann; und
   - n: 0; 1; 2; 3; oder 4;
bedeuten.

Bevorzugt werden in der Komponente (a) Farbbildner der Formel (1) eingesetzt, worin
- R₁: Wasserstoff, Halogen oder C₁-C₄-Alkyl;
- R₂: Nitro; SO₂R₄; SO₂OR₅; SO₂NR₆R₇; COR₈; CONR₆R₇; oder C₁-C₄-Halogenalkyl;
- n: 0, 1, 2, 3 oder 4;
- R₃: wenn n 1,2,3 oder 4 ist, Halogen; wenn n 1 oder 2 ist, C₁-C₄-Alkyl, C₁-C₄-Halogenalkyl; oder wenn n 1 ist, Nitro, COR₈, Amino, mono-C₁-C₄-Alkylamino oder di-C₁-C₄Alkylamino;
- R₄: C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
- R₅: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-alkyl;
- R₆ und R₇: unabhängig voneinander Wasserstoff; oder C₁-C₄-Alkyl; oder
- R₆ und R₇: gemeinsam mit dem Stickstoffatom, an welches sie gebunden sind, einen unsubstituierten oder durch C₁-C₄-Alkyl substituierten Pyrrolidin- oder Piperidin-Ring;
- R₈: Wasserstoff; C₁-C₄-Alkyl; C₁-C₄-Halogenalkyl; C₁-C₄-Alkoxy; unsubstituiertes oder im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy;
- R₉ und R₁₀: unabhängig voneinander C₁-C₈-Alkyl; oder 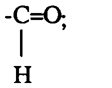
- X₁ und X₂: unabhängig voneinander Wasserstoff; C₁-C₅-Alkyl; oder
- X₁ und X₂: gemeinsam mit dem Stickstoffatom, an welches sie gebunden sind, einen unsubstituierten oder durch C₁-C₄-Alkyl substituierten Pyrrolidin- oder Piperidin-Ring; und
- X₃: Wasserstoff oder Methyl;
bedeutet.

Ganz besonders bevorzugt sind Verbindungen der Formel (1), worin
- R₁: Wasserstoff oder Methyl;
- R₂: Nitro; SO₂R₄; SO₂OR₅; SO₂NR₆R₇; COR₈; CONR₆R₇; oder C₁-C₄-Halogenalkyl;
- n: 0, 1, 2, 3 oder 4;
- R₃: wenn n 1,2,3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
- R₄: C₁-C₄-Alkyl; C₁-C₄-Halogenalkyl; unsubstituiertes oder im Phenylrest durch Halogen , C₁-C₄-Alkyl, oder C₁-C₄-Alkoxy, substituiertes Phenyl;
- R₅: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₂-Alkoxy substituiertes Phenyl oder Phenyl-C₁-C₂-alkyl;
- R₆ und R₇: unabhängig voneinander Wasserstoff; oder C₁-C₄-Alkyl;
- R₈: Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy;
- R₉ und R₁₀: unabhängig voneinander C₁-C₈-Alkyl; oder 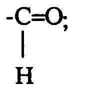
- X₁ und X₂: unabhängig voneinander Wasserstoff; C₁-C₅-Alkyl; oder
- X₁ und X₂: gemeinsam mit dem Stickstoffatom, an welches sie gebunden sind, einen unsubstituierten Pyrrolidin- oder Piperidin-Ring; und
- X₃: Wasserstoff oder Methyl;
bedeutet.

Praktisch wichtige Farbbildner der Formel (1) sind solche Verbindungen, worin
R₂ COR₈ und
R₈ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy bedeutet,
   und insbesondere solche Verbindungen der Formel (1), worin
R₈ C₁-C₄-Alkyl, C₁-C₄-Alkoxy oder Phenyl-C₁-C₂-Alkoxy
bedeutet.

Bevorzugte Farbbildner der Formel (2) sind solche Verbindungen, bei denen R₁₀ C₁-C₈-Alkyl bedeutet.

Bei den Farbbildnern der Formel (3) stehen solche Verbindungen im Vordergrund, bei denen
X₄ und X₅, unabhängig voneinander, unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl;
Y₃ unsubstituiertes oder im Phenylrest durch Halogen, C₁-C₄-Alkyl, C₁-C₄-Halogenalkyl,
C₁-C₄-Alkoxy substituiertes Phenyl; und
   der Benzolring A durch Halogen; C₁-C₅-Alkyl; C₁-C₅-Alkoxy; oder NR₁₃R₁₄ substituiert ist.

Bevorzugte Farbbildner der Komponente (b) sind solche Verbindungen der Formel (4), worin
Y₁ und Y₂ unabhängig voneinander C₃-C₅-Alkyl und
R₉ C₁-C₅-Alkyl oder 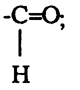 und
R₁₁ Wasserstoff oder C₁-C₅-Alkyl; bedeuten, und insbesondere solche, bei denen
Y₁ und Y₂ unabhängig voneinander C₄-C₅-Alkyl bedeuten.

Ganz besonders bevorzugt sind Verbindungen, bei denen Y₁ und Y₂ die gleiche Bedeutung haben.

Besonders bevorzugtes Farbbildnergemisch enthält als Komponente (a) eine Verbindung der Formel (1), worin
- R₁: Wasserstoff, Halogen oder Methyl;
- R₂: COR₈;
- n: 0, 1, 2, 3 oder 4;
- R₃: wenn n 1,2,3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
- R₈: Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
- X₁ und X₂: unabhängig voneinander Wasserstoff; C₁-C₅-Alkyl; oder
- X₁ und X₂: gemeinsam mit dem Stickstoffatom, an welches sie gebunden sind, einen unsubstituierten Pyrrolidin- oder Piperidin-Ring; und
- X₃: Wasserstoff oder Methyl;
und/oder eine Verbindung der Formel (2), worin
- R₁₀: C₁-C₅-Alkyl bedeuten;
und als Komponente (b) eine Verbindung der Formel (4), worin
- Y₁ und Y₂: C₃-C₅-Alkyl und
- R₉: C₁-C₅-Alkyl; oder 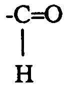 und
- R₁₁: Wasserstoff oder C₁-C₅-Alkyl;
bedeuten.

Die Verbindungen der Formel (1) sind aus der EP-A-0,561,738 bekannt und können nach den dort beschriebenen Methoden hergestellt werden.

Die Verbindungen der Formel (3) sowie deren Herstellung sind aus der US-A-4,675,497 bekannt.

Die Verbindungen der Formel (4) sind zum Teil bekannte Verbindungen, wie z.B. aus der US-A-5,071,480. Zum Teil fallen auch neue Verbindungen darunter. Diese neuen Verbindungen stellen einen weiteren Erfindungsgegenstand dar.

Sie entsprechen der Formel 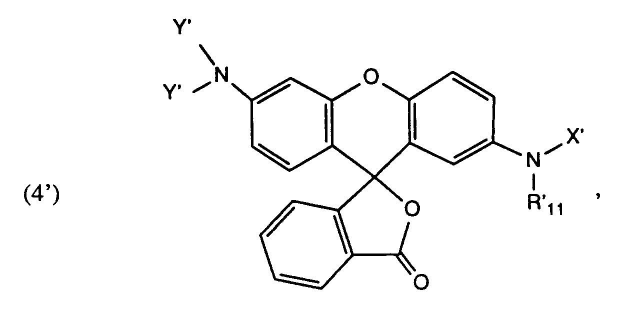 worin
- X': C₁-C₅-Alkyl; oder 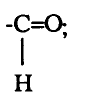
- Y': C₄-C₅-Alkyl; und
- R'₁₁: Wasserstoff oder C₁-C₅-Alkyl;
bedeuten.

Die Verbindungen der Formel (4') können nach an sich bekannten Verfahren hergestellt werden, z.B. dadurch, dass man ein Benzophenon der Formel 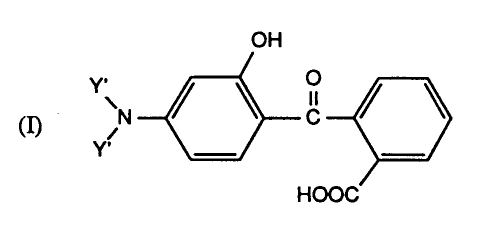 mit einem Phenol oder Phenolether der Formel 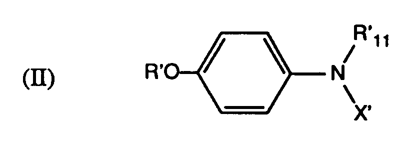 bei Temperaturen von vorzugsweise 0 bis 70° C in 50 bis 100 %iger Schwefelsäure, gegebenenfalls unter Abspaltung der Formylschutzgruppe, zu einem Phthalid der Formel 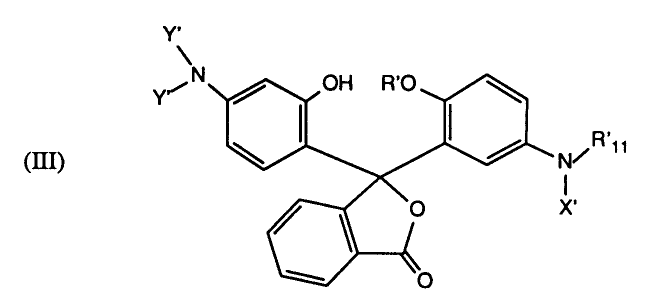 kondensiert und anschliessend bei Temperaturen von 20 bis 100° C zu einer Verbindung der Formel (4') cyclisiert. X', Y' und R'₁₁ haben dabei die in Formel (4') angegebene Bedeutung. R' bedeutet Wasserstoff oder C₁-C₄-Alkyl.

Dieses Herstellungsverfahren stellt einen weiteren Erfindungsgegenstand dar.

Das neue Farbbildnergemisch kann durch einfaches Mischen und Mahlen der genannten Komponenten (a) und (b) hergestellt werden, wobei homogene Pulvergemische erhalten werden, die bei Raumtemperatur lagerstabil sind. Vorzugsweise werden die einzelnen Farbbildnerkomponenten zunächst einzeln in nassem Zustand gemahlen und anschliessend als Dispersion gemischt.

Die Komponenten (a) und (b) werden vorzugsweise in kristalliner Form verwendet.

Die Farbbildnerkomponenten (a) und (b) liegen in der Regel in einem Gewichtsverhältnis von 1:5 bis 1:1 und vorzugsweise 1:3 bis 1:2 vor. Damit können die gewünschten Farbnuancen eingestellt werden.

Das erfindungsgemässe Farbbildnergemisch ist zur Herstellung von druckempfindlichen und insbesondere wärmeempfindlichen Aufzeichnungssystemen sehr gut geeignet. Zu diesem Zweck können die Komponenten (a) und (b) auch getrennt eingesetzt werden.

Das erfindungsgemässe Farbbildnergemisch ist normalerweise farblos oder höchstens schwach gefärbt. Wenn die sublimationsechten Farbbildnergemische mit einem vorzugsweise sauren Entwickler, d.h. einem Elektronenakzeptor, in Kontakt gebracht werden, so ergeben sie intensive graue oder schwarze Farbtöne, die ausgezeichnet lichtecht sind. Sie können auch im Gemisch mit einem oder mehreren anderen bekannten Farbbildnern, wie z.B. 3,3-(Bis-aminophenyl-)-phthaliden, wie CVL, 3-Indolyl-3-aminophenyl- aza- oder -diazaphthaliden, (3,3-Bis-indolyl-)-phthaliden, 3-Aminofluoranen, substituierten 3,7-Diaminofluoranen, Leukoauraminen, Spiropyranen, Spirodipyranen, Chromenopyrazolen, Chromenoindolen, Phenoxazinen, Phenothiazinen, Carbazolylmethanen oder weiteren Triarylmethan-Leukofarbstoffen verwendet werden.

Das erfindungsgemässe Farbbildnergemisch zeigt sowohl auf aktivierten Tonen, wie auch auf phenolischen Unterlagen eine ausgezeichnete Farbintensität und Lichtechtheit. Es eignet sich vor allem als schnell entwickelndes Farbbildnergemisch für die Verwendung in einem druckempfindlichen oder insbesondere wärmeempfindlichen Aufzeichnungsmaterial, das sowohl Kopier- als auch Registriermaterial sein kann. Es zeichnet sich dadurch aus, dass es lagerstabil ist. Schwarze Aufzeichnungen, die mit dem erfindungsgemässen Farbbildnergemisch mit Phenolen erzeugt werden, zeichnen sich gegenüber mit einer einzigen Komponente, wie z.B. 2-Phenylamino-3-methyl-6-diethylaminofluoran erhaltenen Aufzeichnungen durch eine verbesserte Lagerstabilität sowie geringere Hintergrundverfärbung aus.

Ein druckempfindliches Material besteht beispielsweise aus mindestens einem Paar von Blättern, die ein Farbbildnergemisch aus den Komponenten (a) und (b) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxid, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein. Die Zinksalicylate sind z.B. in EP-A-0,181,283 oder DE-A-2,242,250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxid, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Das Farbbildnergemisch liefert an den Punkten, an denen es mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes, mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Das Farbbildnergemisch wird vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester wie z.B. Tricresylphosphat, Di-n-butylphthalat, Dioctylphthalat, Trichlorethylphosphat, aromatische Ether wie Benzylphenylether, Kohlenwasserstofföle, wie Paraffin oder Kerosin, aromatische Kohlenwasserstoffe, z.B. mit Isopropyl, Isobutyl, sek.Butyl oder tert.Butyl alkylierte Derivate von Diphenyl, Naphthalin oder Terphenyl, Dibenzyltoluol; partiell hydriertes Terphenyl, mono-, bis- oder tetra-C₁-C₃-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmäßig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die das Farbbildnergemisch enthaltenden Mikrokapseln können zur Herstellung von druckempfindlichen Kopiermaterialien der verschiedensten bekannten Arten verwendet werden. Die Systeme unterscheiden sich im wesentlichen voneinander durch die Anordnung der Kapseln, der Farbreaktanten und durch das Trägermaterial. Bevorzugt wird eine Anordnung, bei der das eingekapselte Farbbildnergemisch in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, dass die das Farbbildnergemisch enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo-oder-copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Das erfindungsgemässe Farbbildnergemisch kann insbesondere in einem thermoreaktiven Aufzeichnungsmaterial verwendet werden. Dabei zeichnet sich das erfindungsgemässe Gemisch dadurch aus, dass die Komponenten (a) und (b) bei gleicher Temperatur, insbesondere von 110 bis 200°C Farbe entwickeln. Das thermoreaktive Aufzeichnungsmaterial enthält in der Regel mindestens einen Schichtträger, das Farbbildnergemisch, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs-und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass das Farbbildnergemisch in einer Bindemittelschicht gelöst oder dispergiert ist und in einer zweiten Schicht der Entwickler in dem Bindemittel gelöst und dispergiert ist. Eine andere Möglichkeit besteht darin, dass sowohl das Farbbildnergemisch als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren wie sie in druckempfindlichen Papieren verwendet werden. Beispiele für Entwickler sind die bereits erwähnten Tonminerale und Phenolharze oder auch phenolische Verbindungen, wie sie beispielsweise in der DE-PS-1,251,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethyl- oder -benzylester, 4-Hydroxydiphenylsulfon, 4'-Hydroxy-4-methyldiphenylsulfon, 4'-Hydroxy-4-isopropoxydiphenylsulfon, 4,4'-Cyclo-hexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, o-, m-, p-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Zur Herstellung des thermoreaktiven Aufzeichnungsmaterials werden auch schmelzbare, filmbildende Bindemittel verwendet. Diese Bindemittel sind normalerweise wasserlöslich, während die Farbbildner und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Gemisch aus Koreaktant, Aktivator und Farbbildner. Auf diese Weise wird ein Kontakt hergestellt.

Als Aktivatoren kommen z.B. Dimethylterephthalat, Benzyl-Benzyloxy-Benzoat, Terephthalsäuredibenzylester, Phenyl-1-Oxy-2-naphthoat, p-Benzyl-biphenyl, 2,6-Di-tert.Butyl-p-kresol, Salicylsäurephenylester, Benzophenon N-Phenylbenzolsulfonami, Phenyl-1-hydroxy-2-naphthoat oder 4'4-Dibutoxydiphenylsulfon in Betracht. Die Aktivatoren dienen in erter Linie zur Schmelzpunkterniedrigung des Farbbildnergemisches.

Wenn das Farbbildnergemisch und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol verwendet werden. Die bevorzugte Anordnung ist jedoch diejenige, bei der das Farbbildnergemisch und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten z.B. Talk, Titandioxid, Zinkoxid, Aluminiumhydroxid, Calciumcarbonat (z.B. Kreide), Tone oder auch organische Pigmente, wie z.B. Harnstoff-Formaldehydpolymerisate enthalten. Um die Farbbildung nur innerhalb eines begrenzten Temperaturbereiches zu bewirken, können Substanzen wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzyltherephthalat oder andere entsprechende, schmelzbare Produkte, welche das gleichzeitige Schmelzen des Farbbildnergemischs und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Erfindungsgemässe druck- und insbesondere wärmeempfindliche Aufzeichnungsmaterialien zeichnen sich durch hervorragendes Lagerverhalten und eine hohe Papierweisse (Hintergrund) aus.

In den folgenden Herstellungs- und Anwendungsbeispielen beziehen sich die Prozentsätze, wenn nicht anders angegeben, auf das Gewicht.

### Herstellungsbeispiele der neuen Verbindungen:

### Beispiel 1:

55,4 g 4'-Dibutylamino-2-hydroxybenzophenon-2-carbonsäure werden bei 40°C in 217 g 98%iger Schwefelsäure gelöst. Bei ca. 10°C trägt man 21 g N-Methyl-p-anisidin ein und rührt während 20 Stunden bei 20 bis 25°C. Die entstandene Reaktionslösung trägt man auf eine gut rührende Mischung von 700 ml Wasser, 800 ml Toluol und 455 ml einer 10N Natronlauge aus. Man hält eine Stunde unter Rückfluss bei ca. 83°C, trennt bei ca. 70°C die wässrige Phase, wäscht die Toluolphase zweimal mit je 150 ml Wasser und destilliert das Toluol unter vermindertem Druck ab. Man erhält 65,4 g Rohprodukt der Formel 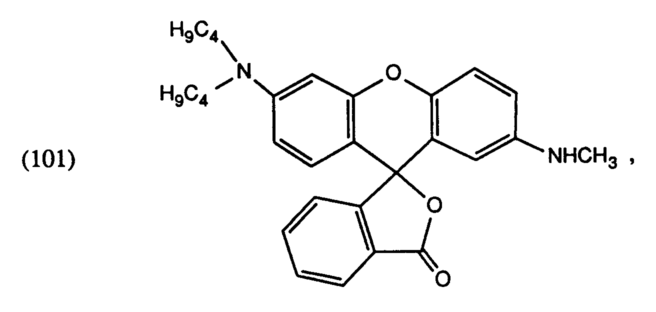 welches nach Umkristallisation aus einem Gemisch aus Isopropylalkohol und Toluol einen Schmelzpunkt von 217-218°C aufweist.

Auf handelsüblichem CF-Papier sowie in der Thermoapplikation erzeugt man damit einen olive-grünen Farbton, auf weissem Hintergrund (Optische Dichte:0,008 Db; (Db= "density black"); die Messung erfolgt mit einem Densitometer GRETAG® SPM100; vgl. auch Beispiel 10).

### Beispiel 2:

22,2 g 3'-Dibutylamino-2-hydroxybenzophenon-2-carbonsäure werden bei ca. 40°C in 87 g Schwefelsäure (98%ig) gelöst. Bei ca. 10°C gibt man innerhalb von 30 Minuten 9,9 g N-Formyl-N-methyl-p-anisidin zu. Die Reaktionsmasse wird während 20 Stunden bei 20-25°C gerührt. Zur Spaltung der Formyl-Schutzgruppe tropft man vorsichtig 40 ml Wasser zu und hält während einer Stunde eine Temperatur von ca. 100°C. Die vorliegende Lösung wird nun auf ein Gemisch von 100 ml Wasser, 400 ml Toluol und 182 ml Natronlauge 10N gegeben (pH-Wert ca. 10-11) und während einer Stunde unter Rückfluss gehalten. Bei 75°C wird die wässrige Phase abgetrennt. Die Toluolphase wird zweimal mit je 200 ml Wasser von 75°C gewaschen, azeotrop entwässert und klärfiltriert. Durch Abdestillation des Toluols und Kristallisation in Methanol gewinnt man 21,9 g des Produktes der Formel (101), das zu dieser Verbindung identische Eigenschaften aufweist.

### Beispiel 3 bis 9:

Analog zu Beispiel 1 werden die folgenden Verbindungen hergestellt (Tabelle 1):

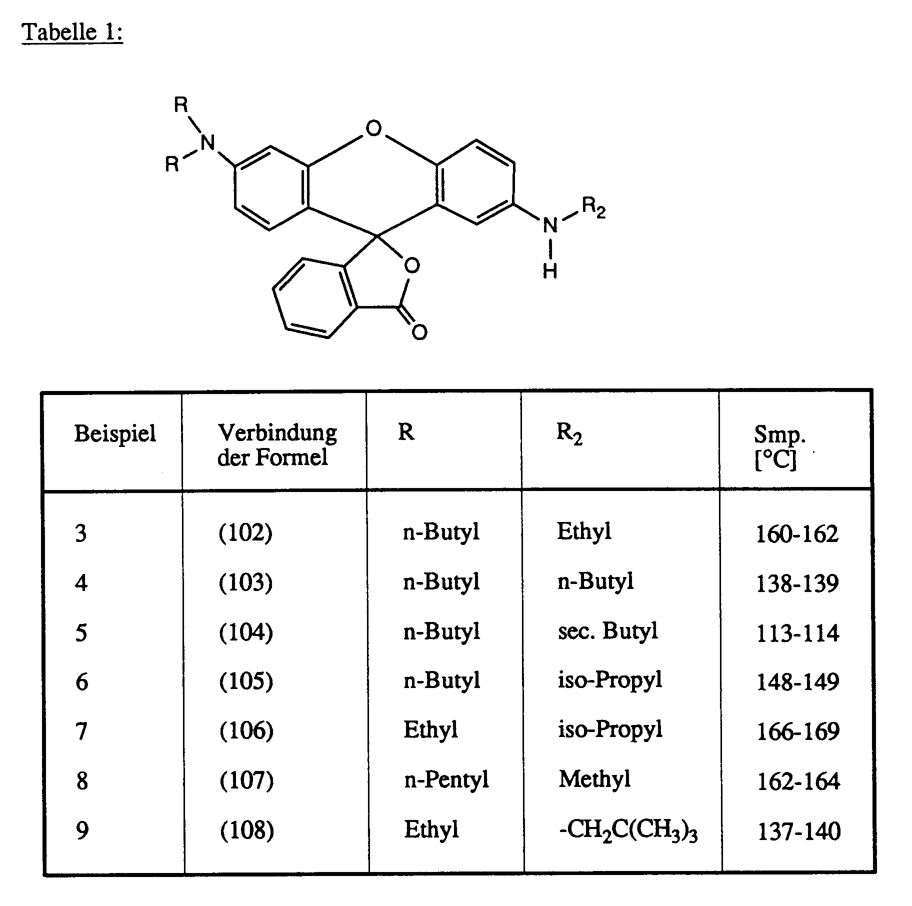

### Beispiel 10a:

73,9 g 3'-Dibutylamino-2-hydroxybenzophenon-2-carbonsäure werden bei max. 40°C in 295 g Schwefelsäure (100%ig) gelöst. Man kühlt auf ca. 10°C und gibt 33 g N-Formyl-N-methyl-p-anisidin in ca. 30 Minuten dazu. Die Reaktionsmasse wird während 20 Stunden bei 20 bis 25°C gerührt und anschliessend auf
1000 ml Wasser und
800 g Eis bei 0 bis 10°C ausgetragen.

Die Suspension wird nach Rühren von 1,5 Stunden bei 10°C abfiltriert und mit 400 ml kaltem Wasser nachgewaschen.

Das feuchte Nutschgut der Formel 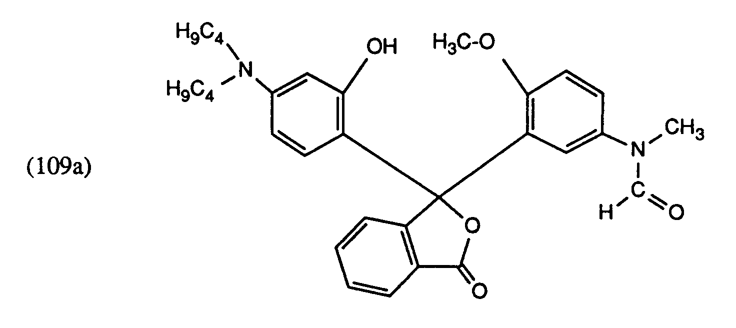 wird in eine Mischung aus
400 ml Toluol und
100 ml Wasser und
53 g Soda eingetragen, auf Rückfluss (ca 81°C) erhitzt und während 7 Stunden bei dieser Temperatur gehalten. Die Toluolphase wird abgetrennt, mit Wasser gewaschen, azeotrop getrocknet, mit Aktivkohle klärfiltriert und eingeengt. Der so erhaltene Rückstand wiegt 50 g und wird aus Isopropylalkohol umkristallisiert.

Man erhält die Fluoranverbindung der Formel 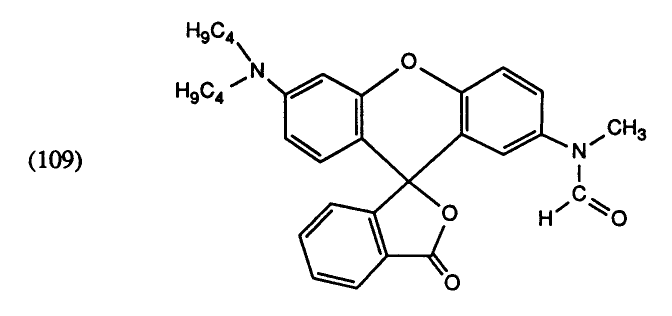 als farblose Kristalle mit einem Schmelzpunkt von 193 bis 194°C.

### Beispiel 10b:

Die Phthalidverbindung der Formel (109a) kann auch durch zweistündiges Kochen in DMF bei 147-149°C zum Fluoran der Formel (109) ringgeschlossen werden.
Ausbeute 95% d.TH.

### Beispiel 11:

20 g der Fluoranverbindung der Formel (101) werden in 50 ml Ameisensäure techn. à 85% Gehalt eingetragen und auf Rückfluss von ca. 97°C erhitzt. Nach 20 Minuten ist die Formylierungsreaktion vollständig und die entstandene rote Lösung wird auf 1 Liter Eiswasser bei ca. 10°C ausgetragen. Man lässt die Temperatur auf 20 bis 25°C steigen, filtriert und wäscht das Produkt auf einer Nutsche. Nach Trocknung im Vakkum bei 70°C erhält man 18,5 g eines schwach rosa gefärbten Produktes der Formel (109) mit einem Schmelzpunkt von 192 bis 194°C.

Auf handlesüblichen CF-Papieren lässt sich damit eine intensive, rote Farbe erzeugen.

In der Thermoapplikation wird ebenfalls eine rote Farbe auf weissem Hintergrund (Dm=0,07: Dm="density magenta") erhalten. Der Lagertest dieses Thermopapiers ist hervorragend: nach einer Stunde bei 58°C und 50% relativer Luftfeuchtigkeit misst man eine Dm = 0,08. Mit dem Faksimile-Gerät erzeugt man eine Dm = 1,23.

### Applikationsbeispiele

### Beispiel 12: Anwendung in der Thermographie

### a) Herstellung der Dispersion (A):

1,3 g des oliven Farbbildners der Formel (101) und
0,7 g des orangen Farbbildners der Formel 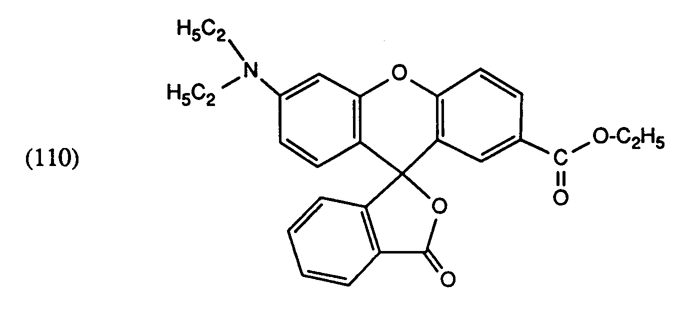 werden in einer Kugelmühle mit 7 g einer wässrigen, 10%igen Lösung von Polyvinylalkohol und 4 g Wasser bei Raumtemperatur auf eine Teilchengrösse von 1-4 µm gemahlen.

### b) Herstellung der Dispersion (B):

2 g 4,4'-Isopropylidendiphenol (Bisphenol A) werden mit 7 g einer wässrigen, 10%igen Lösung von Polyvinylalkohol V03/140 und 4 g Wasser bei Raumtemperatur auf eine Teilchengrösse von 2 bis 4 µm gemahlen.

3 Gewichtsteile der Dispersion (B) und 1 Gewichtsteil der Dispersion (A) werden gut gemischt und anschliessend mit einem Rakel auf ein Papier aufgetragen, so dass nach dem Trocknen an der Luft ein Auftragsgewicht von ca. 0,6 g/m² resultiert. Das Papier wird 24 Stunden bei 23°C und 56% relativer Luftfeuchtigkeit klimatisiert.
Hintergrundfarbe: 0,06 Db (Die Messung des Db-Wertes erfolgt mit einem Densitometer GRETAG® SPM 100).

Durch Beschreiben mit einem handlesüblichen Faksimile-Gerät (INFOTEC ® 6510) wird eine schwarze Schrift mit 1,10 Db erhalten.

Das getrocknete Papier wird während einer Stunde bei 58°C und 50% relativer Luftfeuchtigkeit gelagert. Man erhält einen Db-Wert von 0,10.

### Beispiel 13:

Man verfährt wie in Beispiel 12 beschrieben mit dem Unterschied, dass man die Farbbildnerkomponenten (a) und (b) zunächst einzeln mahlt und dann mischt. Die in Beispiel 12 erhaltenen Ergebnisse können noch übertroffen werden.

### Beispiel 14:

1,5 g des Farbbildners der Formel (101) werden in einer Kugelmühle mit 5,25 g einer wässrigen, 10%igen Lösung von Polyvinylalkohol und 3 g Wasser bei Raumtemperatur auf eine Korngrösse von 1-4 µm gemahlen.

0,5 g der Verbindung der Formel (111) werden in einer zweiten Kugelmühle mit 1,75 g einer wässrigen 10%igen Lösung von Polyvinylalkohol und 1 g Wasser bei Raumtemperatur auf eine Korngrösse von 1-4 µm gemahlen.

Man mischt nun die so erhaltenen gemahlenen Farbbildner (=Dispersion 14A).

Zu einem Gewichtsteil der Dispersion (14A) setzt man 3 Gewichtsteile der Dispersion B aus Beispiel 12 zu und mischt gründlich. Dieses Gemisch wird nun, wie in Beispiel 12 beschrieben, auf ein Papier aufgetragen und ergibt nach dem Trocknen durch Beschreiben mit dem Faksimile-Gerät eine schwarze Schrift mit einer optischen Dichte von 1,15 Db. Der Hintergrund ist weiß mit einer optischen Dichte von 0,07 Db.

### Beispiel 15:

Man verfährt wie in Beispiel 12 beschrieben, verwendet aber anstelle des orangen Farbbildners der Formel (110) 0,7 g 3-Cyclohexyl-amino-6-chlorfluoran zur Herstellung der Dispersion (A).Durch Beschreiben mit dem Faksimile-Gerät erhält man eine schwarze Schrift auf weissem Hintergrund.

### Beispiel 16:

Verfährt man wie in Beispiel 12 beschrieben, verwendet aber eine Mischung von Farbbildnern aus
1,8 Gew. Teilen des Farbbildners der Formel (101),
0,9 Gew. Teilen des Farbbildners der Formel (110) und
0,3 Gew. Teilen des Farbbildners der Formel 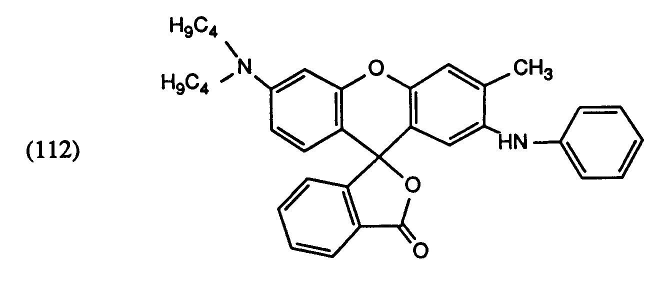 zur Herstellung der Dispersion (A), so erhält man durch Beschreiben mit dem Faksimile-Gerät eine schwarze Schrift auf weissem Hintergrund.

### Beispiel 17:

Verfährt man wie in Beispiel 12 beschrieben, verwendet aber eine Mischung von Farbbildnern aus
1,8 Gew. Teilen des Farbbildners der Formel (101),
0,9 Gew. Teilen des Farbbildners der Formel (110) und
0,3 Gew. Teilen des Farbbildners der Formel 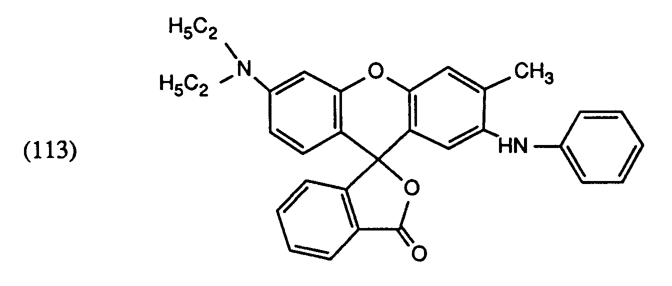 zur Herstellung der Dispersion (A), so erhält man durch Beschreiben mit dem Faksimile-Gerät eine schwarze Schrift auf weissem Hintergrund.

### Beispiel 18:

Man verfährt wie in Beispiel 12 beschrieben, verwendet aber eine Mischung von Farbbildnern aus
1,8 Gew. Teilen des Farbbildners der Formel (101),
0,9 Gew. Teilen des Farbbildners der Formel (110) und
0,3 Gew. Teilen des Farbbildners der Formel 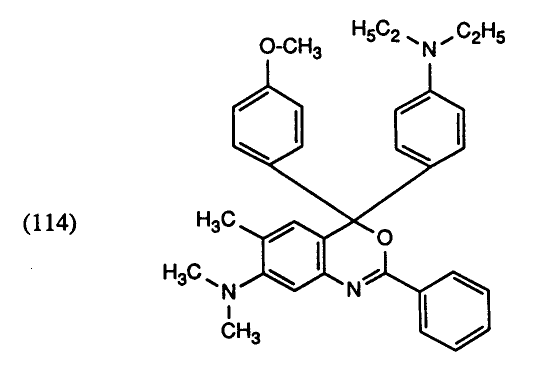 zur Herstellung der Dispersion (A), so erhält man durch Beschreiben mit dem Faksimile-Gerät eine schwarze Schrift auf weissem Hintergrund.

### Beispiel 19:

Anstelle der Dispersion (B) aus Beispiel 12 lässt sich die folgende Grundformulierung, bestehend aus folgenden Komponenten, einsetzen (T = Gewichtsteile):

| | Konz. [%] | Trockenteile [g] |
|---|---|---|
| Koreaktant Bisphenol A oder 2,2,-Bis-4-hydroxy-4-methylpentan* | 33,3 | 7,5 |
| Aktivator Benzylbiphenyl | 25 | 8 |
| MgCO₃* | 25 | 6,8 |
| Kartoffelstärke | 12 | 16,7 |
| Zinkumstearat* | 20 | 2,5 |
| Titandioxid* | 25 | 1,6 |
| Aufheller vom sulfonierten Stilben-Typ | 25 | 0,6 |
| Carboxyliertes Styrol-Butadien-Copolymer | 50 | 2 |
| Deionisiertes Wasser | 100 | 33,14 |

Diese Grundformulierung wird folgendermassen hergestellt:
Die mit (*) bezeichneten Einzelverbindungen werden mit einem handelsüblichen Tensid dispergiert. Anschliessend wird deionisertes Wasser hizugegeben und die restlichen Komponenten hinzugemischt.

### Beispiel 20:

Anstelle der im Beispiel 19 eingesetzten Aktivatoren, Pigmente und Koreaktanden werden Grundformulierungen mit folgenden Einzelverbindungen eingesetzt:
- Pigmente:: CaCO₃; Harnstoff/Formaldehydkondensat
- Koreaktant:: 2,4-Dihydroxybenzophenon;
- Aktivatoren:: Dimethylterephthalat; Benzyl-Benzyloxy-Benzoat; Terephthalsäuredibenzyle ster; Phenyl-1-Oxy-2-naphthoat; p-Benzylbiphenyl; 2,6-Di-tert.Butyl-p-kresol; Salicylsäurephenylester; Benzophenon; N-Phenylbenzolsulfonamide; Phenyl-1-hydroxy-2-naphthoate; 4'4-Dibutoxydiphenylsulfon;.

### Beispiel 21: Applikation auf Durchschreibesysteme

Eine Lösung von 2,2 g der Verbindung der Formel (103), 1,4 g der Verbindung der Formel (110), 0,6 g 3,3-Bis(4-dimethylaminophenyl)-6-dimethylaminophthalid und 0,8 g N-Butylcarbazol-3-yl-bis-(4-N-methyl-N-phenylaminophenyl)-methan in 80 g Diisopropylnaphthalin und 19 g Kerosin wird in an sich bekannter Weise mit Gelantine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste, die sublimationsechten Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive schwarze Kopie, die ausgezeichnet lichtecht ist.

## Patentansprüche

1. Farbbildnergemisch, enthaltend
(a) eine Verbindung der Formel 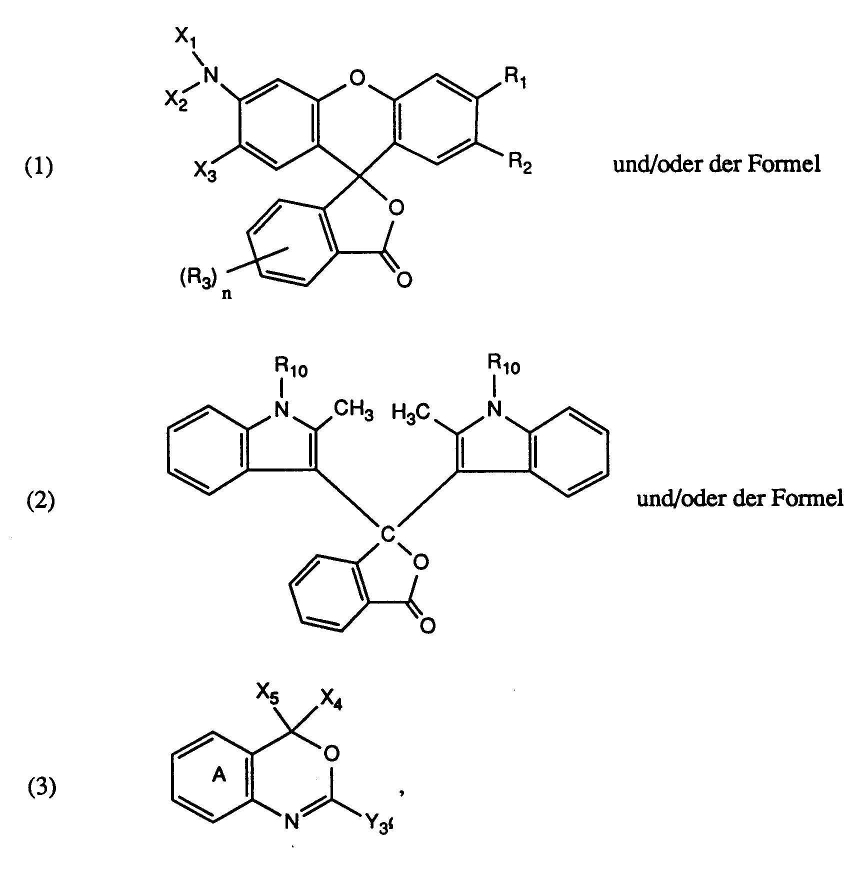 und
(b) eine Verbindung der Formel 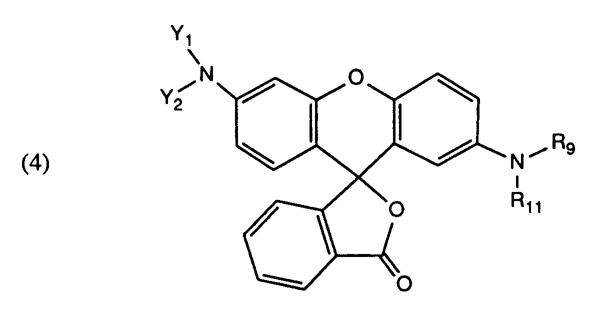 oder ein Farbbildnergemisch, enthaltend mindestens zwei Verbindungen der Komponente (b),
worin
R₁ Wasserstoff, Hydroxy, Halogen oder C₁-C₄-Alkyl;
R₂ Wasserstoff; Nitro; SO₂R₄; SO₂OR₅; SO₂NR₆R₇; COR₈; CONR₆R₇; oder C₁-C₄-Halogenalkyl; nicht substituierter oder durch Halogen oder Hydroxy substituierter 2-Triazinyl- oder 1-Benztriazolylrest;
R₃ Halogen; Nitro; C₁-C₄-Alkyl; C₁-C₄-Halogenalkyl; Amino; mono-C₁-C₄-Alkylamino; di-C₁-C₄-Alkylamino; oder COR₈;
R₄ C₁-C₈-Alkyl; C₁-C₈-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₄-Alkyl;
R₅ Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
R₆ und R₇ unabhängig voneinander Wasserstoff; oder C₁-C₈-Alkyl; oder
R₆ und R₇ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₈ Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
R₉ und R₁₀ unabhängig voneinander C₁-C₈-Alkyl; oder 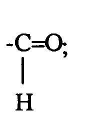
R₁₁ Wasserstoff oder C₁-C₅-Alkyl;
R₁₂ und R₁₃, unabhängig voneinander, je Wasserstoff, unsubstituiertes oder durch Halogen, Hydroxy, Cyano oder C₁-C₅-Alkoxy substituiertes Alkyl mit höchstens 12 Kohlenstoffatomen, C₅-C₇-Cycloalkyl oder unsubstituiertes oder durch Halogen, Cyano, C₁-C₅-Alkyl oder C₁-C₅-Alkoxy ringsubstituiertes Phenylalkyl oder Phenyl oder
R₁₂ und R₁₃ zusammen mit dem sie verbindenden Stickstoffatom einen fünf-oder sechsgliedrigen heterocyclischen Rest;
X₁ und X₂, unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄-Alkyl; 2-Tetrahydrofuranyl, oder
X₁ und X₂ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
X₃ Wasserstoff oder C₁-C₄-Alkyl;
X₄ und X₅ unabhängig voneinander unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl oder Phenyl-C₁-C₄-Alkyl; einen 2-Pyrrolyl-, 2-Thienyl-, 2- oder 3-Indolyl-, 2-Benzofuranyl- oder 2-Naphthothienylrest;
Y₁ und Y₂ unabhängig voneinander C₁-C₅-Alkyl;
Y₃ Wasserstoff; C₁-C₅-Alkyl; C₅-C₇-Cycloalkyl, unsubstituiertes oder im phenylrest durch Halogen, C₁-C₄-Alkyl, C₁-C₄-Halogenalkyl, C₁-C₄-Alkoxy substituiertes Phenyl;
der Benzolring A durch Halogen; Cyano; Nitro; C₁-C₅-Alkyl; C₁-C₅-Alkoxy; C₁-C₅-Alkylthio; C₁-C₅-Alkylcarbonyl; C₁-C₅-Alkoxycarbonyl; oder NR₁₂R₁₃ substituiert sein kann; und
n 0; 1; 2; 3; oder 4;
bedeuten.

2. Farbbildnergemisch nach Anspruch 1, enthaltend
(a) eine Verbindung der Formel 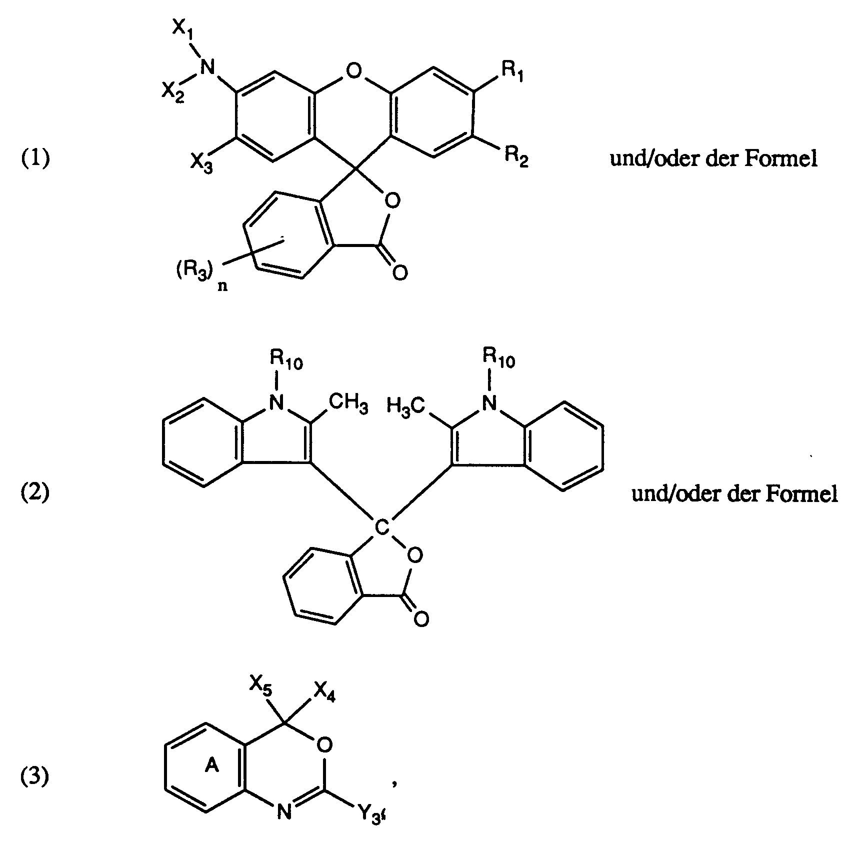 und
(b) eine Verbindung der Formel 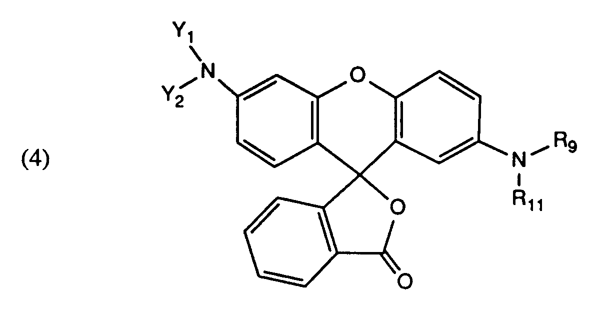 worin
R₁ Wasserstoff, Hydroxy, Halogen oder C₁-C₄-Alkyl;
R₂ Wasserstoff; Nitro; SO₂R₄; SO₂OR₅; SO₂NR₆R₇; COR₈; CONR₆R₇; oder C₁-C₄-Halogenalkyl; nicht substituierter oder durch Halogen oder Hydroxy substituierter 2-Triazinyl- oder 1-Benztriazolylrest;
R₃ Halogen; Nitro; C₁-C₄-Alkyl; C₁-C₄-Halogenalkyl; Amino; mono-C₁-C₄-Alkylamino; di-C₁-C₄-Alkylamino; oder COR₈;
R₄ C₁-C₈-Alkyl; C₁-C₈-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₄-Alkyl;
R₅ Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
R₆ und R₇ unabhängig voneinander Wasserstoff; oder C₁-C₈-Alkyl; oder
R₆ und R₇ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₈ Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
R₉ und R₁₀ unabhängig voneinander C₁-C₈-Alkyl; oder 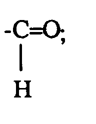
R₁₁ Wasserstoff oder C₁-C₅-Alkyl;
R₁₂ und R₁₃, unabhängig voneinander, je Wasserstoff, unsubstituiertes oder durch Halogen, Hydroxy, Cyano oder C₁-C₅-Alkoxy substituiertes Alkyl mit höchstens 12 Kohlenstoffatomen, C₅-C₇-Cycloylkyl oder unsubstituiertes oder durch Halogen, Cyano, C₁-C₅-Alkyl oder C₁-C₅-Alkoxy ringsubstituiertes Phenylalkyl oder Phenyl oder
R₁₂ und R₁₃ zusammen mit dem sie verbindenden Stickstoffatom einen fünf- oder sechsgliedrigen heterocyclischen Rest;
X₁ und X₂, unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄-Alkyl; 2-Tetrahydrofuranyl, oder
X₁ und X₂ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
X₃ Wasserstoff oder C₁-C₄-Alkyl;
X₄ und X₅ unabhängig voneinander unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl oder Phenyl-C₁-C₄-Alkyl; einen 2-Pyrrolyl-, 2-Thienyl-, 2- oder 3-Indolyl-, 2-Benzofuranyl- oder 2-Naphthothienylrest;
Y₁ und Y₂ unabhängig voneinander C₁-C₅-Alkyl;
Y₃ Wasserstoff; C₁-C₅-Alkyl; C₅-C₇-Cycloalkyl, unsubstituiertes oder im Phenylrest durch Halogen, C₁-C₄-Alkyl, C₁-C₄-Halogenalkyl, C₁-C₄-Alkoxy substituiertes Phenyl;
der Benzolring A durch Halogen; Cyano; Nitro; C₁-C₅-Alkyl; C₁-C₅-Alkoxy; C₁-C₅-Alkylthio; C₁-C₅-Alkylcarbonyl; C₁-C₅-Alkoxycarbonyl; oder NR₁₂R₁₃ substituiert sein kann; und
n 0; 1; 2; 3; oder 4;
bedeuten.

3. Farbbildnergemisch nach Anspruch 1 oder 2, dadurch gekennzeichnet, dass in Formel (1)
R₁ Wasserstoff, Halogen oder C₁-C₄-Alkyl;
R₂ Nitro; SO₂R₄; SO₂OR₅; SO₂NR₆R₇; COR₈; CONR₆R₇; oder C₁-C₄-Halogenalkyl;
n 0, 1, 2, 3 oder 4;
R₃ wenn n 1,2,3 oder 4 ist, Halogen; wenn n 1 oder 2 ist, C₁-C₄-Alkyl, C₁-C₄-Halogenalkyl; oder wenn n 1 ist, Nitro, COR₈, Amino, mono-C₁-C₄-Alkylamino oder di-C₁-C₄Alkylamino;
R₄ C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
R₅ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-Alkyl;
R₆ und R₇ unabhängig voneinander Wasserstoff; oder C₁-C₄-Alkyl; oder
R₆ und R₇ gemeinsam mit dem Stickstoffatom, an welches sie gebunden sind, einen unsubstituierten oder durch C₁-C₄-Alkyl substituierten Pyrrolidin- oder Piperidin-Ring;
R₈ Wasserstoff; C₁-C₄-Alkyl; C₁-C₄-Halogenalkyl; C₁-C₄-Alkoxy; unsubstituiertes oder im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy;
R₉ und R₁₀ unabhängig voneinander C₁-C₈-Alkyl; oder 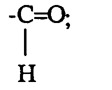
X₁ und X₂ unabhängig voneinander Wasserstoff; C₁-C₅-Alkyl; oder
X₁ und X₂ gemeinsam mit dem Stickstoffatom, an welches sie gebunden sind, einen unsubstituierten oder durch C₁-C₄-Alkyl substituierten Pyrrolidin- oder Piperidin-Ring; und
X₃ Wasserstoff oder Methyl;
bedeutet.

4. Farbbildnergemisch nach einem der Ansprüche 1 bis 3, dadurch gekennzeichnet, dass in Formel (1)
R₁ Wasserstoff oder Methyl;
R₂ Nitro; SO₂R₄; SO₂OR₅; SO₂NR₆R₇; COR₈; CONR₆R₇; oder C₁-C₄-Halogenalkyl;
n 0, 1, 2, 3 oder 4;
R₃ wenn n 1,2,3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
R₄ C₁-C₄-Alkyl; C₁-C₄-Halogenalkyl; unsubstituiertes oder im Phenylrest durch Halogen , C₁-C₄-Alkyl, oder C₁-C₄-Alkoxy, substituiertes Phenyl;
R₅ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl oder Phenyl-C₁-C₂-Alkyl;
R₆ und R₇ unabhängig voneinander Wasserstoff; oder C₁-C₄-Alkyl;
R₈ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy;
R₉ und R₁₀ unabhängig voneinander C₁-C₈-Alkyl; oder 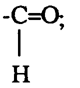
X₁ und X₂ unabhängig voneinander Wasserstoff; C₁-C₅-Alkyl; oder
X₁ und X₂ gemeinsam mit dem Stickstoffatom, an welches sie gebunden sind, einen unsubstituierten Pyrrolidin- oder Piperidin-Ring; und
X₃ Wasserstoff oder Methyl;
bedeutet.

5. Farbbildnergemisch nach einem der Ansprüche 1 bis 4, enthaltend einen Farbbildner der Formel (1), worin
R₂ COR₈,
R₈ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy bedeutet.
R₂ COR₈ und
R₈ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy bedeutet.

6. Farbbildnergemisch nach einem der Ansprüche 1 bis 5, dadurch gekennzeichnet, dass als Farbbildner der Formel (2) Verbindungen verwendet werden, worin R₁₀ C₁-C₈-Alkyl bedeutet.

7. Farbbildnergemisch nach einem der Ansprüche 1 bis 6, dadurch gekennzeichnet, dass als Farbbildner Verbindungen der Formel (3) verwendet werden, worin
X₄ und X₅, unabhängig voneinander, unsubstituiertes oder im Phenylrest durch Halogen, Nitro, C₁-C₄-Alkyl, C₁-C₄-Alkoxy, C₁-C₄-Halogenalkyl oder -NR₁₂R₁₃ substituiertes Phenyl;
Y₃ unsubstituiertes oder im Phenylrest durch Halogen, C₁-C₄-Alkyl, C₁-C₄-Halogenalkyl, C₁-C₄-Alkoxy substituiertes Phenyl; bedeuten und
der Benzolring A durch Halogen; C₁-C₅-Alkyl; C₁-C₅-Alkoxy; oder NR₁₃R₁₄ substituiert ist.

8. Farbbildnergemisch nach einem der Ansprüche 1 bis 7, enthaltend als Komponente (b) eine Verbindung der Formel (4), worin
Y₁ und Y₂ unabhängig voneinander C₃-C₅-Alkyl und
R₉ C₁-C₅-Alkyl oder 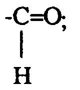 und
R₁₁ Wasserstoff oder C₁-C₅-Alkyl;
bedeuten.

9. Farbbildnergemisch nach Anspruch 8, enthaltend einen Farbbildner (b) der Formel (4), worin
Y₁ und Y₂ C₄-C₅-Alkyl bedeutet.

10. Farbbildnergemisch nach Anspruch 8 oder 9, dadurch gekennzeichnet, dass Y₁ und Y₂ die gleiche Bedeutung haben.

11. Farbbildnergemisch nach Anspruch 1, enthaltend als Komponente (a) eine Verbindung der Formel (1), worin
R₁ Wasserstoff, Halogen oder Methyl;
R₂ COR₈;
n 0, 1, 2, 3 oder 4;
R₃ wenn n 1,2,3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
R₈ Wasserstoff; C₁-C₄-Alkyl; C₁-C₄-Alkoxy; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy;
X₁ und X₂ unabhängig voneinander Wasserstoff; C₁-C₅-Alkyl; oder
X₁ und X₂ gemeinsam mit dem Stickstoffatom, an welches sie gebunden sind, einen unsubstituierten Pyrrolidin- oder Piperidin-Ring; und
X₃ Wasserstoff oder Methyl;
und/oder eine Verbindung der Formel (2), worin
R₁₀ unabhängig voneinander C₁-C₅-Alkyl bedeuten, und als Komponente (b) eine Verbindung der Formel (4), worin
Y₁ und Y₂ C₃-C₅-Alkyl und
R₉ C₁-C₅-Alkyl; oder 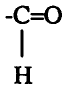 und
R₁₁ Wasserstoff oder C₁-C₅-Alkyl;
bedeuten.

12. Farbbildnergemisch nach einem der Ansprüche 1 bis 11, dadurch gekennzeichnet, dass das Gewichtsverhältnis von (a):(b) 1:5 bis 1:1 beträgt.

13. Farbbildnergemisch nach einem der Ansprüche 1 bis 12, dadurch gekennzeichnet, dass die Komponenten (a) und (b) in kristallinem Zustand vorliegen.

14. Mikroverkapselte Farbbildnergemischlösung, welche eine Verbindung der Formel (1) und/oder der Formel (2), und/oder der Formel (3) und eine Verbindung der Formel (4) oder ein Farbbildnergemisch, enthaltend mindestens zwei Verbindungen der Komponente (b) nach Anspruch 1 enthält.

15. Druckempfindliches Aufzeichungsmaterial enthaltend ein Farbbildnergemisch nach einem der Ansprüche 1 bis 13.

16. Wärmeempfindliches Aufzeichnungsmaterial enthaltend ein Farbbildnergemisch nach einem der Ansprüche 1 bis 13.

17. Wärmeempfindliches Aufzeichnungsmaterial nach Anspruch 16, welches eine wärmeempfindliche Schicht aufweist, die einen Farbbildner und einen Farbentwickler für einen Farbbildner enthält, dadurch gekennzeichnet, dass der Farbbildner ein Farbbildnergemisch nach einem der Ansprüche 1 bis 11 enthält.

18. Verbindungen der Formel 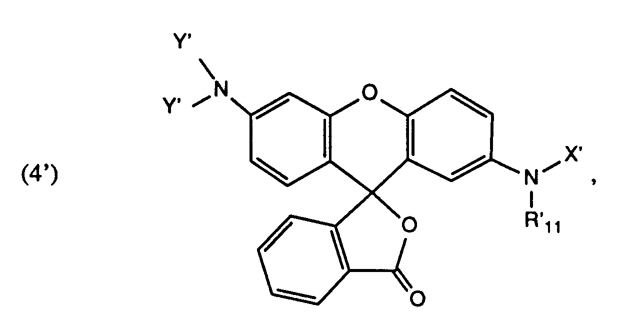 worin
X' C₁-C₅-Alkyl; oder 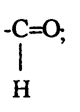
Y' C₄-C₅-Alkyl; und
R'₁₁ Wasserstoff oder C₁-C₅-Alkyl
bedeuten.

19. Verfahren zur Herstellung der Verbindungen der Formel (4'), dadurch gekennzeichnet, dass man ein Benzophenon der Formel 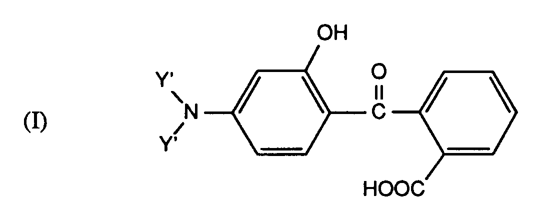 mit einem Phenol oder Phenolether der Formel 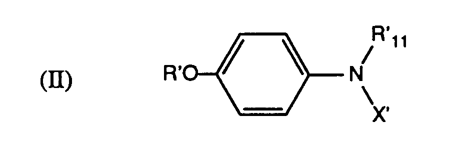 bei Temperaturen von vorzugsweise 0 bis 70° C in 50 bis 100 %iger Schwefelsäure, gegebenenfalls unter Abspaltung der Formylschutzgruppe, zu einem Phthalid der Formel 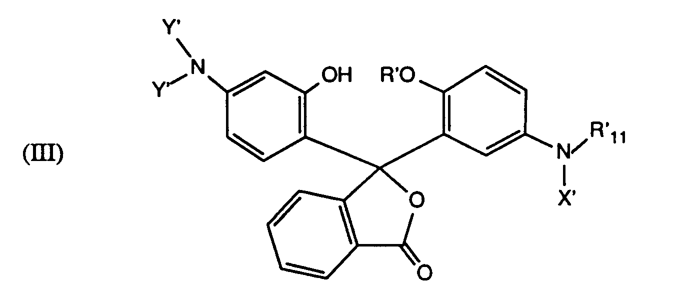 kondensiert und anschliessend bei Temperaturen von 20 bis 100° C zu einer Verbindung der Formel (4') cyclisiert,
worin
X' und Y'und R'₁₁ die in Formel (4') angegebene Bedeutung haben und
R' Wasserstoff oder C₁-C₄-Alkyl
bedeuten.

## Claims

1. A colour former mixture comprising
(a) a compound of formula 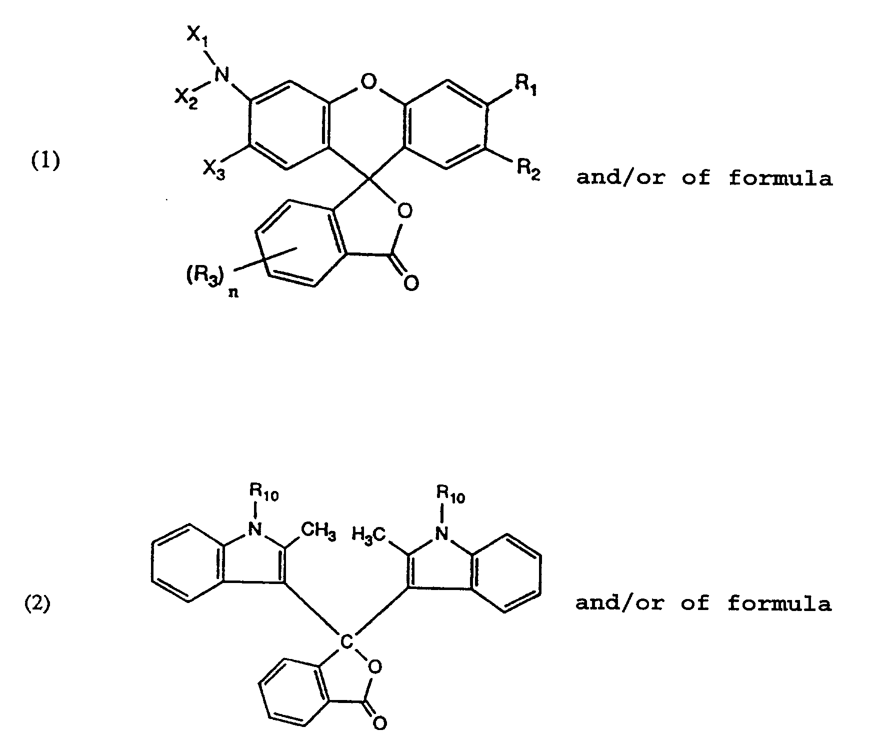 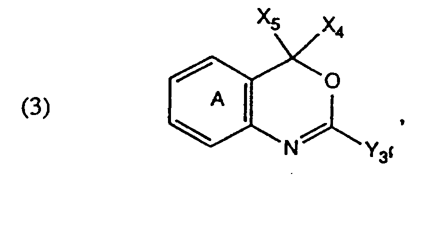 and
(b) a compound of formula 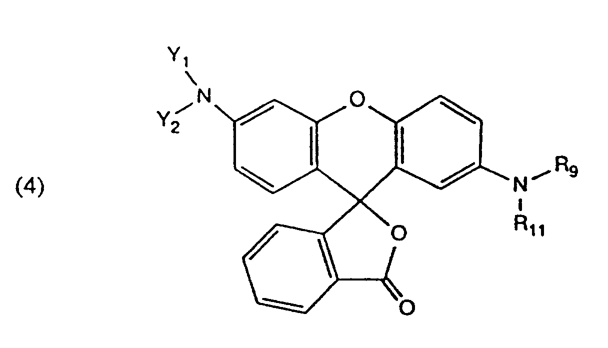 or a colour former mixture comprising at least two compounds of component (b),
in which
R₁ is hydrogen, hydroxy, halogen or C₁-C₄alkyl;
R₂ is hydrogen; nitro; SO₂R₄; SO₂OR₅; SO₂NR₆R₇; COR₈; CONR₆R₇; or C₁-C₄haloalkyl; an unsubstituted or halogen-or hydroxy-substituted 2-triazinyl or 1-benzotriazolyl radical;
R₃ is halogen; nitro; C₁-C₄alkyl; C₁-C₄haloalkyl; amino; mono-C₁-C₄alkylamino; di-C₁-C₄alkylamino; or COR₈;
R₄ is C₁-C₈alkyl; C₁-C₈haloalkyl; phenyl or phenyl-C₁-C₄alkyl, each unsubstituted or substituted in the phenyl moiety by halogen, C₁-C₄alkyl, C₁-C₄haloalkyl or C₁-C₄alkoxy;
R₅ is hydrogen; C₁-C₈alkyl; C₁-C₈haloalkyl; phenyl or phenyl-C₁-C₄alkyl, each unsubstituted or substituted in the phenyl moiety by halogen, C₁-C₄alkyl, C₁-C₄haloalkyl or C₁-C₄alkoxy;
R₆ and R₇ are each independently of the other hydrogen; or C₁-C₈alkyl; or
R₆ and R₇, together with the linking nitrogen atom, are an
unsubstituted or C₁-C₄alkyl-substituted pyrrolidino, piperidino, morpholino, thiomorpholino or piperazino ring;
R₈ is hydrogen; hydroxy; C₁-C₈alkyl; C₁-C₈alkoxy, C₁-C₈haloalkyl; phenyl which is unsubstituted or substituted by halogen, C₁-C₄alkyl, C₁-C₄haloalkyl or C₁-C₄alkoxy; phenyl-C₁-C₄alkyl or phenyl-C₁-C₄alkoxy;
R₉ and R₁₀ are each independently of the other C₁-C₈alkyl;
or 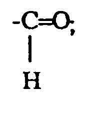
R₁₁ is hydrogen or C₁-C₅alkyl;
R₁₂ and R₁₃ are each independently of the other hydrogen, alkyl of not more than 12 carbon atoms which is unsubstituted or substituted by halogen, hydroxy, cyano or C₁-C₅alkoxy; C₅-C₇cycloalkyl, or phenyl or phenylalkyl which is unsubstituted or ring-substituted by halogen, cyano, C₁-C₅alkyl or C₁-C₅alkoxy, or
R₁₂ and R₁₃, together with the linking nitrogen atom, are a five- or six-membered heterocyclic radical;
X₁ and X₂ are each independently of the other hydrogen; C₁-C₈alkyl; C₄-C₇cycloalkyl which is unsubstituted or substituted by C₁-C₄alkyl or halogen; unsubstituted phenyl or phenyl which is substituted by C₁-C₄alkyl, hydroxy or halogen; phenyl-C₁-C₄alkyl; C₃-C₆alkenyl; C₁-C₄alkoxy; C₁-C₄alkoxy-C₁-C₄alkyl; 2-tetrahydrofuranyl, or
X₁ and X₂, together with the linking nitrogen atom, are an
unsubstituted or C₁-C₄alkyl-substituted pyrrolidine, piperidino, morpholino, thiomorpholino or piperazino ring;
X₃ is hydrogen or C₁-C₄alkyl;
X₄ and X₅ are each independently of the other phenyl or phenyl-C₁-C₄alkyl, each unsubstituted or substituted in the phenyl moiety by halogen, nitro, C₁-C₄alkyl, C₁-C₄alkoxy, C₁-C₄haloalkyl or -NR₁₂R₁₃; a 2-pyrrolyl, 2-thienyl, 2- or 3-indolyl, 2-benzofuranyl or 2-naphthothienyl radical;
Y₁ and Y₂ are each independently of the other C₁-C₅alkyl;
Y₃ is hydrogen; C₁-C₅alkyl; C₅-C₇cycloalkyl, unsubstituted phenyl or phenyl which is substituted by halogen, C₁-C₄alkyl, C₁-C₄haloalkyl or C₁-C₄alkoxy;
the benzene ring A may be substituted by halogen; cyano; nitro; C₁-C₅alkyl; C₁-C₅alkoxy; C₁-C₅alkylthio; C₁-C₅alkylcarbonyl; C₁-C₅alkoxycarbonyl; or NR₁₂R₁₃; and
n is 0; 1; 2; 3; or 4.

2. A colour former mixture according to claim 1, comprising
(a) a compound of formula 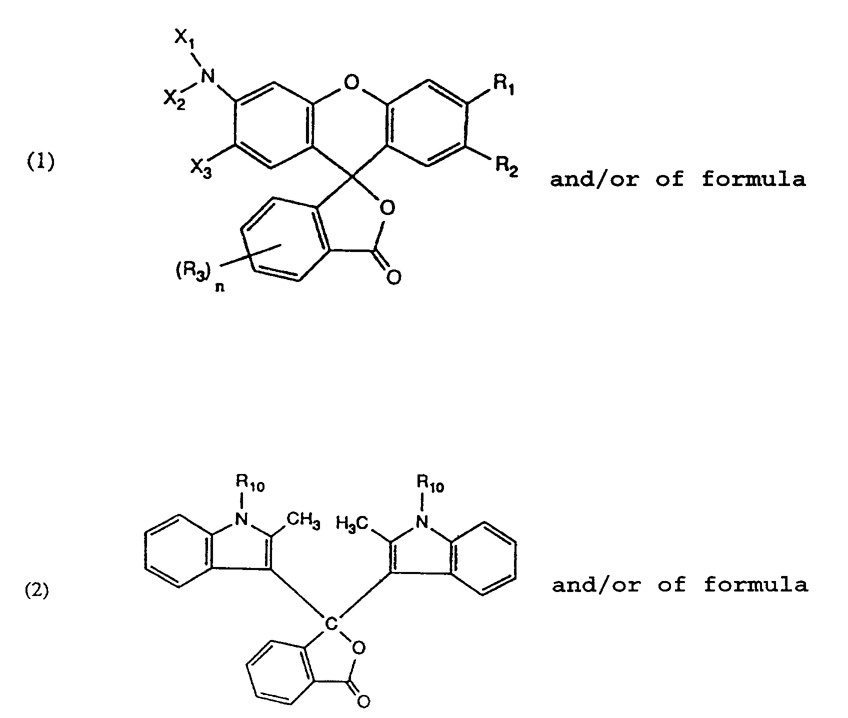 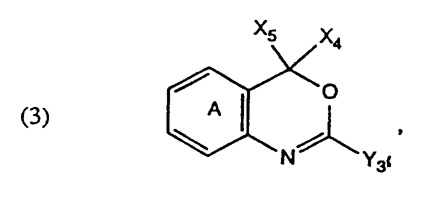 and
(b) a compound of formula 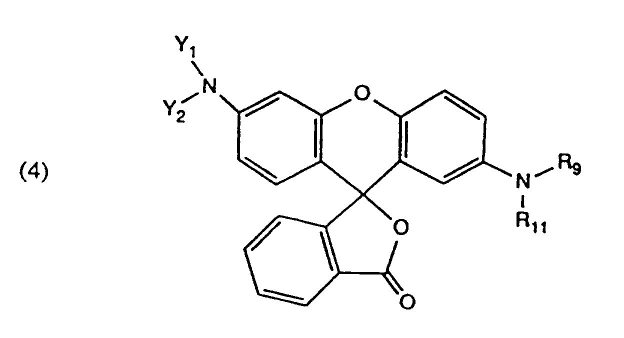 in which
R₁ is hydrogen, hydroxy, halogen or C₁-C₄alkyl;
R₂ is hydrogen; nitro; SO₂R₄; SO₂OR₅; SO₂NR₆R₇; COR₈; CONR₆R₇; or C₁-C₄haloalkyl; an unsubstituted or halogen-or hydroxy-substituted 2-triazinyl or 1-benzotriazolyl radical;
R₃ is halogen; nitro; C₁-C₄alkyl; C₁-C₄haloalkyl; amino; mono-C₁-C₄alkylamino; di-C₁-C₄alkylamino; or COR₈;
R₄ is C₁-C₈alkyl; C₁-C₈haloalkyl; phenyl or phenyl-C₁-C₄alkyl, each unsubstituted or substituted in the phenyl moiety by halogen, C₁-C₄alkyl, C₁-C₄haloalkyl or C₁-C₄alkoxy;
R₅ is hydrogen; C₁-C₈alkyl; C₁-C₈haloalkyl; phenyl or phenyl-C₁-C₄alkyl, each unsubstituted or substituted in the phenyl moiety by halogen, C₁-C₄alkyl, C₁-C₄haloalkyl or C₁-C₄alkoxy;
R₆ and R₇ are each independently of the other hydrogen; or C₁-C₈alkyl; or
R₆ and R₇, together with the linking nitrogen atom, are an unsubstituted or C₁-C₄alkyl-substituted pyrrolidino, piperidino, morpholino, thiomorpholino or piperazino ring;
R₈ is hydrogen; hydroxy; C₁-C₈alkyl; C₁-C₈alkoxy, C₁-C₈haloalkyl; unsubstituted phenyl or phenyl which is substituted by halogen, C₁-C₄alkyl, C₁-C₄haloalkyl or C₁-C₄alkoxy; phenyl-C₁-C₄alkyl or phenyl-C₁-C₄alkoxy;
R₉ and R₁₀ are each independently of the other C₁-C₈alkyl; or 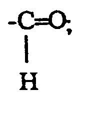
R₁₁ is hydrogen or C₁-C₅alkyl;
R₁₂ and R₁₃ are each independently of the other hydrogen, alkyl of not more than 12 carbon atoms which is unsubstituted or substituted by halogen, hydroxy, cyano or C₁-C₅alkoxy; C₅-C₇cycloalkyl, or phenyl or phenylalkyl which is unsubstituted or ring-substituted by halogen, cyano, C₁-C₅alkyl or C₁C₅-alkoxy, or
R₁₂ and R₁₃, together with the linking nitrogen atom, are a five- or six-membered heterocyclic radical;
X₁ and X₂ are each independently of the other hydrogen; C₁-C₈alkyl; C₄-C₇cycloalkyl which is unsubstituted or substituted by C₁-C₄alkyl or halogen; unsubstituted phenyl or phenyl which is substituted by C₁-C₄alkyl, hydroxy or halogen; phenyl-C₁-C₄alkyl; C₃-C₆alkenyl; C₁-C₄alkoxy; C₁-C₄alkoxy-C₁-C₄alkyl; 2-tetrahydrofuranyl, or
X₁ and X₂, together with the linking nitrogen atom, are an
unsubstituted or C₁-C₄alkyl-substituted pyrrolidino, piperidino, morpholino, thiomorpholino or piperazino ring;
X₃ is hydrogen or C₁-C₄alkyl;
X₄ and X₅ are each independently of the other phenyl or phenyl-C₁-C₄alkyl, each unsubstituted or substituted in the phenyl moiety by halogen, nitro, C₁-C₄alkyl, C₁-C₄alkoxy, C₁-C₄haloalkyl or -NR₁₂R₁₃; a 2-pyrrolyl, 2-thienyl, 2- or 3-indolyl, 2-benzofuranyl or 2-naphthothienyl radical;
Y₁ and Y₂ are each independently of the other C₁-C₅alkyl;
Y₃ is hydrogen; C₁-C₅alkyl; C₅-C₇cycloalkyl, unsubstituted phenyl or phenyl which is substituted by halogen, C₁-C₄alkyl, C₁-C₄haloalkyl or C₁-C₄alkoxy;
the benzene ring A may be substituted by halogen; cyano; nitro; C₁-C₅alkyl; C₁-C₅alkoxy; C₁-C₅alkylthio; C₁-C₅alkylcarbonyl; C₁-C₅alkoxycarbonyl; or NR₁₂R₁₃; and
n is 0; 1; 2; 3; or 4.

3. A colour former mixture according to claim 1 or 2, wherein in formula (1)
R₁ is hydrogen, halogen or C₁-C₄alkyl;
R₂ is nitro; SO₂R₄; SO₂OR₅; SO₂NR₆R₇; COR₈; CONR₆R₇; or C₁-C₄haloalkyl;
n is 0, 1, 2, 3 or 4;
R₃ if n = 1, 2, 3 or 4, is halogen; if n = 1 or 2, is C₁-C₄alkyl or C₁-C₄haloalkyl; or, if n = 1, is nitro, COR₈, amino, mono-C₁-C₄alkylamino or di-C₁-C₄alkylamino;
R₄ is C₁-C₄alkyl; C₁-C₄haloalkyl; phenyl or phenyl-C₁-C₂alkyl, each unsubstituted or substituted in the phenyl moiety by halogen, C₁-C₄alkyl, C₁-C₄haloalkyl or C₁-C₄alkoxy;
R₅ is hydrogen; C₁-C₄alkyl; C₁-C₄haloalkyl; phenyl or phenyl-C₁-C₂alkyl, each unsubstituted or substituted in the phenyl moiety by halogen, C₁-C₄alkyl, C₁-C₄haloalkyl or C₁-C₄alkoxy;
R₆ and R₇ are each independently of the other hydrogen; or C₁-C₄alkyl; or
R₆ and R₇, together with the linking nitrogen atom, are an
unsubstituted or C₁-C₄alkyl-substituted pyrrolidino or piperidino ring;
R₈ is hydrogen; C₁-C₄alkyl; C₁-C₄haloalkyl; C₁-C₄alkoxy; unsubstituted phenyl or phenyl which is substituted by halogen, C₁-C₄alkyl, C₁-C₄haloalkyl or C₁-C₄alkoxy; phenyl-C₁-C₂alkyl or phenyl-C₁-C₂alkoxy;
R₉ and R₁₀ are each independently of the other C₁-C₈alkyl; or 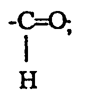
X₁ and X₂ are each independently of the other hydrogen; C₁-C₅alkyl; or
X₁ and X₂, together with the linking nitrogen atom, are an
unsubstituted or C₁-C₄alkyl-substituted pyrrolidino or piperidino ring; and
X₃ is hydrogen or methyl.

4. A colour former mixture according to one of claims 1 to 3, wherein in formula (1)
R₁ is hydrogen or methyl;
R₂ is nitro; SO₂R₄; SO₂OR₅; SO₂NR₆R₇; COR₈; CONR₆R₇; or C₁-C₄haloalkyl;
n is 0, 1, 2, 3 or 4;
R₃ if n = 1, 2, 3 or 4, is halogen; if n = 1 or 2, is methyl; or, if n = 1, is nitro, amino, monoC₁-C₄alkylamino or di-C₁-C₄alkylamino;
R₄ is C₁-C₄alkyl; C₁-C₄haloalkyl; unsubstituted phenyl or phenyl which is substituted by halogen, C₁-C₄alkyl or C₁-C₄alkoxy;
R₅ is hydrogen; C₁-C₄alkyl; C₁-C₄haloalkyl; phenyl or phenyl-C₁-C₂alkyl, each unsubstituted or substituted in the phenyl moiety by halogen, C₁-C₄alkyl, C₁-C₄haloalkyl or C₁-C₄alkoxy;
R₆ and R₇ are each independently of the other hydrogen; or C₁-C₄alkyl;
R₈ is hydrogen; C₁-C₄alkyl; C₁-C₈alkoxy; unsubstituted phenyl or phenyl which is substituted by halogen, C₁-C₄alkyl, C₁-C₄haloalkyl or C₁-C₄alkoxy; phenyl-C₁-C₂alkyl or phenyl-C₁-C₂alkoxy;
R₉ and R₁₀ are each independently of the other C₁-C₈alkyl; or 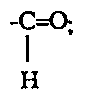
X₁ and X₂ are each independently of the other hydrogen; C₁-C₅alkyl; or
X₁ and X₂, together with the linking nitrogen atom, are an
unsubstituted pyrrolidino or piperidino ring; and
X₃ is hydrogen or methyl.

5. A colour former mixture according to one of claims 1 to 4, comprising a colour former of formula (1), in which
R₂ is COR₈,
R₈ is hydrogen; C₁-C₄alkyl; C₁-C₄alkoxy; unsubstituted phenyl or phenyl which is substituted by halogen, C₁-C₄alkyl, C₁-C₄haloalkyl or C₁-C₄alkoxy; phenyl-C₁-C₂alkyl or phenyl-C₁-C₂alkoxy.
R₂ is COR₈ and
R₈ is hydrogen; C₁-C₄alkyl; C₁-C₄alkoxy; phenyl which is unsubstituted or substituted by halogen, C₁-C₄alkyl, C₁-C₄haloalkyl or C₁-C₄alkoxy; phenyl-C₁-C₂alkyl or phenyl-C₁-C₂alkoxy.

6. A colour former mixture according to one of claims 1 to 5, wherein the colour former of formula (2) used is a compound in which R₁₀ is C₁-C₈alkyl.

7. A colour former mixture according to one of claims 1 to 6, wherein the colour former used is a compound of formula (3) in which
X₄ and X₅ are each independently of the other unsubstituted phenyl or phenyl which is substituted by halogen, nitro, C₁-C₄alkyl, C₁-C₄alkoxy, C₁-C₄haloalkyl or -NR₁₂R₁₃;
Y₃ is unsubstituted phenyl or phenyl which is substituted by halogen, C₁-C₄alkyl, C₁-C₄haloalkyl or C₁-C₄alkoxy; and the benzene ring A is substituted by halogen; C₁-C₅alkyl;
C₁-C₅alkoxy; or NR₁₃R₁₄.

8. A colour former mixture according to one of claims 1 to 7, comprising as component (b) a compound of formula (4) in which
Y₁ and Y₂ are each independently of the other C₃-C₅alkyl, and
R₉ is C₁-C₅alkyl or 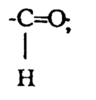 and
R₁₁ is hydrogen or C₁-C₅alkyl.

9. A colour former mixture according to claim 8, comprising a colour former (b) of formula (4) in which Y₁ and Y₂ are C₄-C₅alkyl.

10. A colour former mixture according to claim 8 or 9, wherein Y₁ and Y₂ are identical.

11. A colour former mixture according to claim 1, comprising as component (a) a compound of formula (1) in which
R₁ is hydrogen, halogen or methyl;
R₂ is COR₈;
n is 0, 1, 2, 3 or 4;
R₃ if n = 1, 2, 3 or 4, is halogen; if n = 1 or 2, is methyl; or, if n = 1, is nitro, amino, mono-C₁-C₄alkylamino or di-C₁-C₄alkylamino;
R₈ is hydrogen; C₁-C₄alkyl; C₁-C₄alkoxy; unsubstituted phenyl or phenyl which is substituted by halogen, C₁-C₄alkyl, C₁-C₄haloalkyl or C₁-C₄alkoxy; phenyl-C₁-C₂alkyl or phenyl-C₁-C₂alkoxy;
X₁ and X₂ are each independently of the other hydrogen; C₁-C₅alkyl; or
X₁ and X₂, together with the linking nitrogen atom, are an
unsubstituted pyrrolidino or piperidino ring; and
X₃ is hydrogen or methyl;
and/or a compound of formula (2) in which
R₁₀ are each independently of the other C₁-C₅alkyl, and as component (b) a compound of formula (4) in which
Y₁ and Y₂ are C₃-C₅alkyl and
R₉ is C₁-C₅alkyl; or 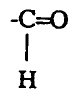 and
R₁₁ is hydrogen or C₁-C₅alkyl.

12. A colour former mixture according to one of claims 1 to 11, wherein the weight ratio of (a):(b) is 1:5 to 1:1.

13. A colour former mixture according to one of claims 1 to 12, wherein components (a) and (b) are in the crystalline state.

14. A microencapsulated solution of a colour former mixture comprising a compound of formula (1) and/or of formula (2), and/or of formula (3) and a compound of formula (4) or a colour former mixture comprising at least two compounds of component (b) according to claim 1.

15. A pressure-sensitive recording material comprising a colour former mixture according to one of claims 1 to 13.

16. A heat-sensitive recording material comprising a colour former mixture according to one of claims 1 to 13.

17. A heat-sensitive recording material according to claim 16, having a heat-sensitive layer comprising a colour former and a colour developer for a colour former, wherein said colour former comprises a colour former mixture according to one of claims 1 to 11.

18. A compound of formula 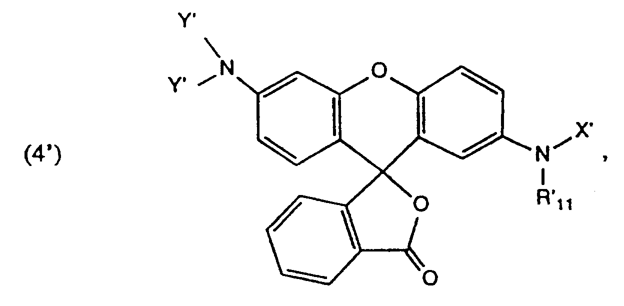 in which
X' is C₁-C₅alkyl; or 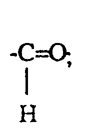
Y' is C₄-C₅alkyl; and
R'₁₁ is hydrogen or C₁-C₅alkyl.

19. A process for the preparation of a compound of formula (4'), which comprises condensing a benzophenone of formula 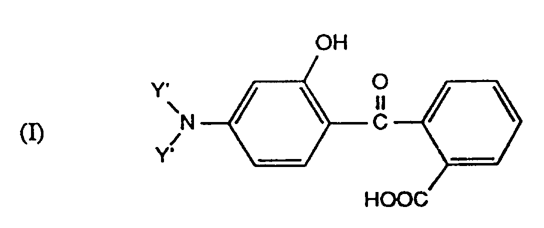 with a phenol or phenol ether of formula 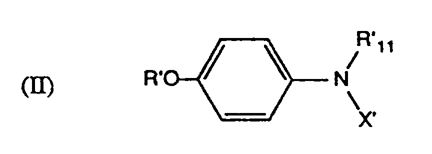 at temperatures from preferably 0 to 70°C in 50 to 100 % sulfuric acid, with removal of the formyl protective group if present, to give a phthalide of formula 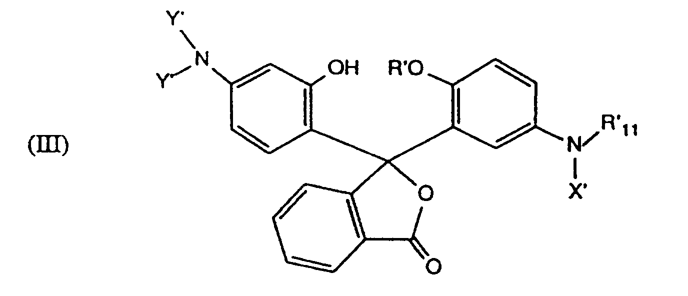 and then cyclising said phthalide of formula (III) at temperatures from 20 to 100°C, to give a compound of formula (4'), in which
X' and Y' and R'₁₁ have the meaning indicated in formula (4'), and
R' is hydrogen or C₁-C₄alkyl.

## Revendications

1. Mélange de formateurs de couleur, contenant
(a) un composé de formule 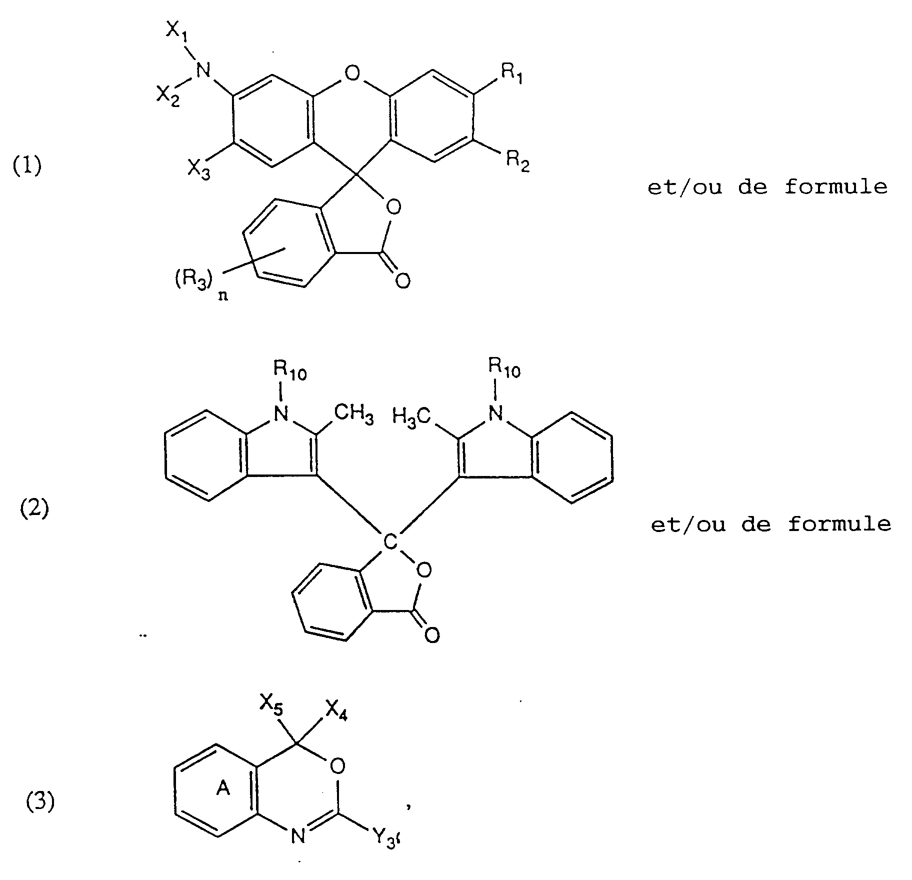 et
(b) un composé de formule 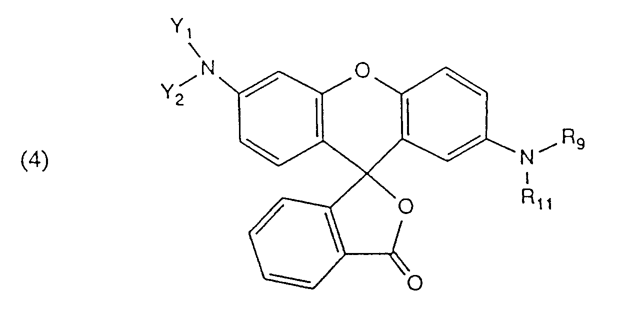 ou encore mélange de formateurs de couleur contenant au moins deux composés du constituant (b),
où
R₁ est un atome d'hydrogène ou un groupe hydroxy, halogéno ou alkyle en C₁-C₄ ;
R₂ est un hydrogène ; le groupe nitro ; SO₂R₄ ; SO₂OR₅ ; SO₂NR₆R₇ ; COR₈ ; CONR₆R₇ ; ou un groupe halogénalkyle en C₁-C₄ ; ou un résidu 2-triazinyle ou 1-benzotriazolyle, non-substitué ou substitué par des substituants halogéno ou hydroxy ;
R₃ est un atome d'halogène ; le groupe nitro ; un groupe alkyle en C₁-C₄ ; halogénalkyle en C₁-C₄ ; amino ; monoalkylamino en C₁-C₄, di-(alkyle en C₁-C₄)amino ; ou COR₈ ;
R₄ est un groupe alkyle en C₁-C₈ ; halogénalkyle en C₁-C₈ ; phényle, ou phényl(alkyle en C₁-C₄) non-substitué, ou substitué sur le noyau phényle par des substituants halogéno, alkyle en C₁-C₄, halogénalkyle en C₁-C₄ ou alcoxy en C₁-C₄ ;
R₅ est un atome d'hydrogène ; un groupe alkyle en C₁-C₈ ; halogénalkyle en C₁-C₈ ; phényle ou phényl(alkyle en C₁-C₄) non-substitué ou substitué sur le noyau phényle par des substituants halogéno, alkyle en C₁-C₄, halogénalkyle en C₁-C₄, alcoxy en C₁-C₄ ;
R₆ et R₇ sont chacun indépendamment de l'autre un atome d'hydrogène ou un groupe alkyle en C₁-C₈ ; ou bien
R₆ et R₇, avec l'atome d'azote auquel ils sont lies, forment un noyau pyrrolidinyle, pipéridinyle, morpholinyle, thiomorpholinyle ou pipérazinyle, non-substitué ou substitué par des substituants alkyle en C₁-C₄ ;
R₈ est un atome d'hydrogène ; le groupe hydroxy ; un groupe alkyle en C₁-C₈ ; alcoxy en C₁-C₈ ; halogénalkyle en C₁-C₈ ; phényle, phényl(alkyle en C₁-C₄) ou phényl(alcoxy en C₁-C₄) non-substitue ou substitue sur le noyau phényle par des substituants halogéno, alkyle en C₁-C₄, halogénalkyle en C₁-C₄, alcoxy en C₁-C₄ ;
R₉ et R₁₀ sont chacun indépendamment de l'autre un groupe alkyle en C₁-C₈ ou -C(H)=O- ;
R₁₁ est un atome d'hydrogène ou un groupe alkyle en C₁-C₅ ;
R₁₂ et R₁₃ sont chacun indépendamment de l'autre un atome d'hydrogène, un groupe alkyle ayant au plus 12 atomes de carbone, non-substitué ou substitué par des substituants halogéno, hydroxy, cyano ou alcoxy en C₁-C₅, cycloalkyle en C₅-C₇, ou encore phénylalkyle ou phényle substitué sur le noyau par des substituants halogéno, cyano, alkyle en C₁-C₅ ou alcoxy en C₁-C₅, ou bien
R₁₂ et R₁₃, avec l'atome d'azote qui les relie, forment un radical hetérocyclique à 5 ou 6 chaînons ;
X₁ et X₂ sont chacun indépendamment de l'autre un atome d'hydrogène ; un groupe alkyle en C₁-C₈ ; un groupe cycloalkyle en C₄-C₇ non-substitué ou substitué par des substituants alkyle en C₁-C₄ ou halogéno ; phényle non-substitué ou substitué par des substituants alkyle en C₁-C₄, hydroxy ou halogéno ; phényl(alkyle en C₁-C₄) ; alcényle en C₃-C₆ ; alcoxy en C₁-C₄ ; (alcoxy en C₁-C₄)(alkyle en C₁-C₄) ; 2-tétrahydrofurannyle, ou encore
X₁ et X₂, avec l'atome d'azote auquel ils sont liés, forment un noyau pyrrolidinyle, pipéridinyle, morpholinyle, thiomorpholinyle ou pipérazinyle non-substitué ou substitué par des substituants alkyle en C₁-C₄ ;
X₃ est un atome d'hydrogène ou un groupe alkyle en C₁-C₄ ;
X₄ et X₅ sont chacun indépendamment de l'autre un groupe phényle ou phényl(alkyle en C₁-C₄) non-substitué ou substitué sur le noyau phényle par des substituants halogéno, nitro, alkyle en C₁-C₄, alcoxy en C₁-C₄, halogénalkyle en C₁-C₄ ou -NR₁₂R₁₃ ; un résidu 2-pyrrolyle, 2-thiényle, 2- ou 3-indolyle, 2-benzofurannyle ou 2-naphtothiényle ;
Y₁ et Y₂ sont chacun indépendamment de l'autre un groupe alkyle en C₁-C₅ ;
Y₃ est un atome d'hydrogène ; un groupe alkyle en C₁-C₅ ; cycloalkyle en C₅-C₇, phényle non-substitué ou substitué sur le noyau phényle par des substituants halogéno, alkyle en C₁-C₄, halogénalkyle en C₁-C₄, alcoxy en C₁-C₄ ;
le noyau benzène A peut être substitué par des substituants halogéno ; cyano ; nitro, alkyle en C₁-C₅ ; alcoxy en C₁-C₅ ; alkylthio en C₁-C₅ ; (alkyle en C₁-C₅)carbonyle ; (alcoxy en C₁-C₅)carbonyle ou NR₁₂R₁₃ ; et
n vaut 0 ; 1 ; 2 ; 3 ; ou 4.

2. Mélange de formateurs de couleur selon la revendication 1, contenant
(a) un composé de formule 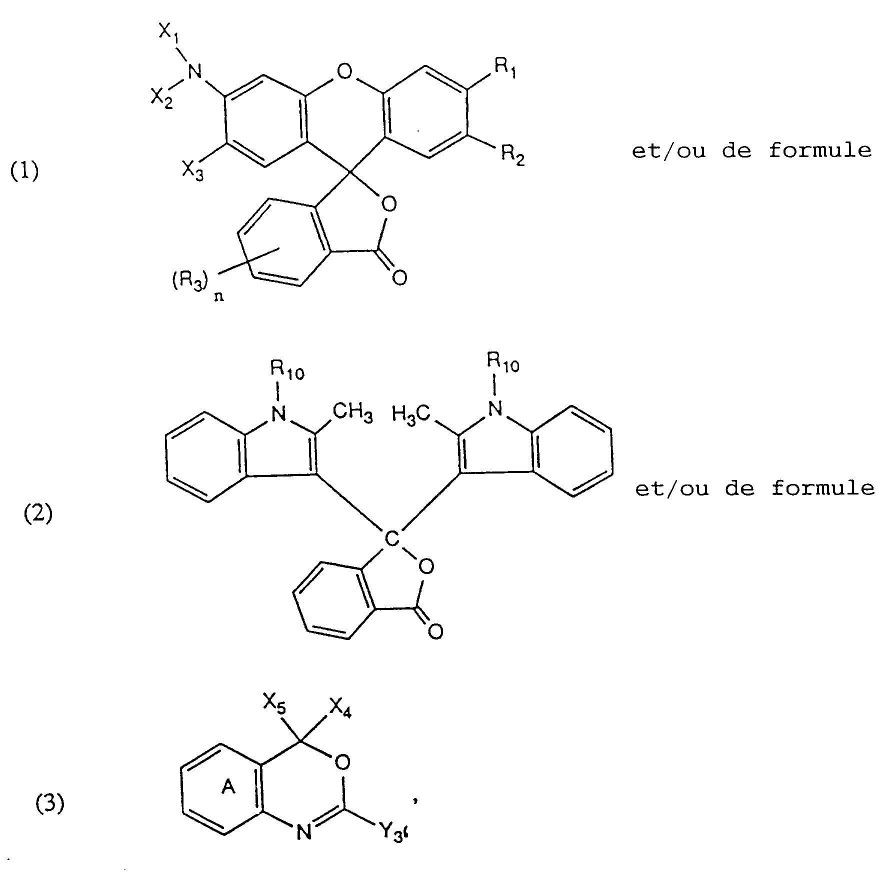 et
(b) un composé de formule 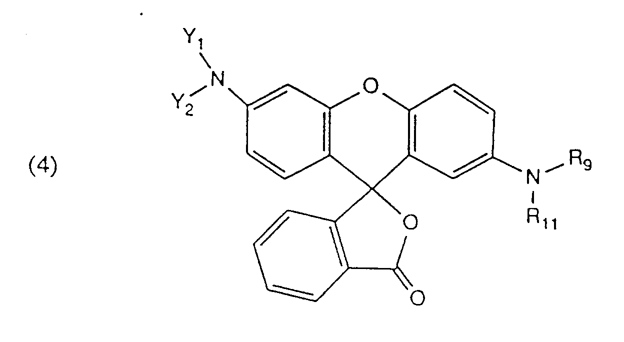 où
R₁ est un atome d'hydrogène ou un groupe hydroxy, halogéno ou alkyle en C₁-C₄ ;
R₂ est un hydrogène ; le groupe nitro ; SO₂R₄ ; SO₂OR₅ ; SO₂NR₆R₇ ; COR₈ ; CONR₆R₇ ; ou un groupe halogénalkyle en C₁-C₄ ; ou un résidu 2-triazinyle ou 1-benzotriazolyle, non-substitué ou substitué par des substituants halogéno ou hydroxy ;
R₃ est un atome d'halogène ; le groupe nitro ; un groupe alkyle en C₁-C₄ ; halogénalkyle en C₁-C₄ ; amino ; monoalkylamino en C₁-C₄, di-(alkyle en C₁-C₄)amino ; ou COR₈ ;
R₄ est un groupe alkyle en C₁-C₈ ; halogénalkyle en C₁-C₈ ; phényle, ou phényl(alkyle en C₁-C₄) non-substitué, ou substitué sur le noyau phényle par des substituants halogéno, alkyle en C₁-C₄, halogénalkyle en C₁-C₄ ou alcoxy en C₁-C₄ ;
R₅ est un atome d'hydrogène ; un groupe alkyle en C₁-C₈ ; halogénalkyle en C₁-C₈ ; phényle ou phényl(alkyle en C₁-C₄) non-substitué ou substitué sur le noyau phényle par des substituants halogéno, alkyle en C₁-C₄, halogénalkyle en C₁-C₄, alcoxy en C₁-C₄ ;
R₆ et R₇ sont chacun indépendamment de l'autre un atome d'hydrogène ou un groupe alkyle en C₁-C₈ ; ou bien
R₆ et R₇, avec l'atome d'azote auquel ils sont liés, forment un noyau pyrrolidinyle, pipéridinyle, morpholinyle, thiomorpholinyle ou pipérazinyle, non-substitué ou substitué par des substituants alkyle en C₁-C₄ ;
R₈ est un atome d'hydrogène ; le groupe hydroxy ; un groupe alkyle en C₁-C₈ ; alcoxy en C₁-C₈ ; halogénalkyle en C₁-C₈ ; phényle, phényl(alkyle en C₁-C₄) ou phényl(alcoxy en C₁-C₄) non-substitué ou substitué sur le noyau phényle par des substituants halogéno, alkyle en C₁-C₄, halogénalkyle en C₁-C₄, alcoxy en C₁-C₄ ;
R₉ et R₁₀ sont chacun indépendamment de l'autre un groupe alkyle en C₁-C₈ ou -C(H)=O- ;
R₁₁ est un atome d'hydrogène ou un groupe alkyle en C₁-C₅ ;
R₁₂ et R₁₃ sont chacun indépendamment de l'autre un atome d'hydrogène, un groupe alkyle ayant au plus 12 atomes de carbone, non-substitué ou substitué par des substituants halogéno, hydroxy, cyano ou alcoxy en C₁-C₅, cycloalkyle en C₅-C₇, ou encore phénylalkyle ou phényle substitué sur le noyau par des substituants halogéno, cyano, alkyle en C₁-C₅ ou alcoxy en C₁-C₅, ou bien
R₁₂ et R₁₃, avec l'atome d'azote qui les relie, forment un radical hétérocyclique à 5 ou 6 chaînons ;
X₁ et X₂ sont chacun indépendamment de l'autre un atome d'hydrogène ; un groupe alkyle en C₁-C₈ ; un groupe cycloalkyle en C₄-C₇ non-substitué ou substitué par des substituants alkyle en C₁-C₄ ou halogéno ; phényle non-substitué ou substitué par des substituants alkyle en C₁-C₄, hydroxy ou halogéno ; phényl(alkyle en C₁-C₄) ; alcényle en C₃-C₆ ; alcoxy en C₁-C₄ ; (alcoxy en C₁-C₄)(alkyle en C₁-C₄) ; 2-tétrahydrofurannyle, ou encore
X₁ et X₂, avec l'atome d'azote auquel ils sont liés, forment un noyau pyrrolidinyle, pipéridinyle, morpholinyle, thiomorpholinyle ou pipérazinyle non-substitué ou substitué par des substituants alkyle en C₁-C₄ ;
X₃ est un atome d'hydrogène ou un groupe alkyle en C₁-C₄ ;
X₄ et X₅ sont chacun indépendamment de l'autre un groupe phényle ou phényl(alkyle en C₁-C₄) non-substitué ou substitué sur le noyau phényle par des substituants halogéno, nitro, alkyle en C₁-C₄, alcoxy en C₁-C₄, halogénalkyle en C₁-C₄ ou -NR₁₂R₁₃ ; un résidu 2-pyrrolyle, 2-thiényle, 2- ou 3-indolyle, 2-benzofurannyle ou 2-naphtothiényle ;
Y₁ et Y₂ sont chacun indépendamment de l'autre un groupe alkyle en C₁-C₅ ;
Y₃ est un atome d'hydrogène ; un groupe alkyle en C₁-C₅ ; cycloalkyle en C₅-C₇, phényle non-substitué ou substitué sur le noyau phényle par des substituants halogéno, alkyle en C₁-C₄, halogénalkyle en C₁-C₄, alcoxy en C₁-C₄ ;
le noyau benzène A peut être substitué par des substituants halogéno ; cyano ; nitro, alkyle en C₁-C₅ ; alcoxy en C₁-C₅ ; alkylthio en C₁-C₅ ; (alkyle en C₁-C₅)carbonyle ; (alcoxy en C₁-C₅)carbonyle ou NR₁₂R₁₃ ; et
n vaut 0 ; 1 ; 2 ; 3 ; ou 4.

3. Mélange de formateurs de couleur selon la revendication 1 ou 2, caractérisé en ce que, dans la formule (1) :
R₁ est un atome d'hydrogène, un atome d'halogène ou un groupe alkyle en C₁-C₄ ;
R₂ est le groupe nitro ; SO₂R₄ ; SO₂OR₅ ; SO₂NR₆R₇ ; COR₈ ; CONR₆R₇ ; ou un groupe halogénalkyle en C₁-C₄ ;
n vaut 0, 1, 2, 3 ou 4 ;
R₃, quand n vaut 1, 2, 3 ou 4, est un atome d'halogène ; quand n vaut 1 ou 2, un groupe alkyle en C₁-C₄, halogénalkyle en C₁-C₄ ; ou encore quand n vaut 1, un groupe nitro, COR₈, amino, mono(alkyle en C₁-C₄)amino ou di(alkyle en C₁-C₄)amino ;
R₄ est un groupe alkyle en C₁-C₄ ; halogénalkyle en C₁-C₄ ; phényle ou phényl(alkyle en C₁-C₂) non-substitué, ou substitué sur le noyau phényle par des substituants halogéno, alkyle en C₁-C₄, halogénalkyle en C₁-C₄, alcoxy en C₁-C₄ ;
R₅ est un atome d'hydrogène ; un groupe alkyle en C₁-C₄ ; halogénalkyle en C₁-C₄ ; phényle ou phényl(alkyle en C₁-C₂) non-substitué, ou substitué sur le noyau phényle par des substituants halogéno, alkyle en C₁-C₄, halogénalkyle en C₁-C₄, alcoxy en C₁-C₄ ;
R₆ et R₇ sont chacun indépendamment de l'autre un atome d'hydrogène ou un groupe alkyle en C₁-C₄ ; ou bien
R₆ et R₇, avec l'atome d'azote auquel ils sont liés, forment un noyau pyrrolidinyle ou pipéridinyle non-substitué ou substitué par des substituants alkyle en C₁-C₄ ;
R₈ est un atome d'hydrogène ; un groupe alkyle en C₁-C₄ ; halogénalkyle en C₁-C₄ ; alcoxy en C₁-C₄ ; phényle non-substitué ou substitué sur le noyau phényle par des substituants halogéno, alkyle en C₁-C₄, halogénalkyle en C₁-C₄, alcoxy en C₁-C₄ ; phényl(alkyle en C₁-C₂) ou phényl(alcoxy en C₁-C₂) ;
R₉ et R₁₀ sont chacun indépendamment de l'autre un groupe alkyle en C₁-C₈ ou -C(H)=O- ;
X₁ et X₂ sont chacun indépendamment de l'autre un atome d'hydrogène ou un groupe alkyle en C₁-C₅ ; ou bien
X₁ et X₂, avec l'atome d'azote auquel ils sont liés, forment un noyau pyrrolidinyle ou pipéridinyle non-substitué ou substitué par des substituants alkyle en C₁-C₄ ; et
X₃ est un atome d'hydrogène ou le groupe méthyle.

4. Mélange de formateurs de couleur selon l'une des revendications 1 à 3, caractérisé en ce que, dans la formule (1) :
R₁ est un atome d'hydrogène ou le groupe méthyle ;
R₂ est le groupe nitro ; SO₂R₄ ; SO₂OR₅ ; SO₂NR₆R₇ ; COR₈ ; CONR₆R₇ ; ou un groupe halogénalkyle en C₁-C₄ ;
n vaut 0, 1, 2, 3 ou 4 ;
R₃, quand n vaut 1, 2, 3 ou 4, est un atome d'halogène ; quand n vaut 1 ou 2, un groupe méthyle ; ou quand n vaut 1, un groupe nitro, amino, mono(alkyle en C₁-C₄)amino ou di(alkyle en C₁-C₄)amino ;
R₄ est un groupe alkyle en C₁-C₄ ; halogénalkyle en C₁-C₄ ; phényle non-substitué ou substitué sur le noyau phényle par des substituants halogéno, alkyle en C₁-C₄ ou alcoxy en C₁-C₄ ;
R₅ est un atome d'hydrogène ; un groupe alkyle en C₁-C₄ ; halogénalkyle en C₁-C₄ ; phényle ou phényl(alkyle en C₁-C₂) non-substitué ou substitué sur le noyau phényle par des substituants halogéno, alkyle en C₁-C₄, halogénalkyle en C₁-C₄, alcoxy en C₁-C₂ ;
R₆ et R₇ sont chacun indépendamment de l'autre un atome d'hydrogène ou un groupe alkyle en C₁-C₄ ;
R₈ est un atome d'hydrogène un groupe alkyle en C₁-C₄ ; alcoxy en C₁-C₄ ; phényle non-substitué ou substitué sur le noyau phényle par des substituants halogéno, alkyle en C₁-C₄, halogénalkyle en C₁-C₄ ou alcoxy en C₁-C₄ ; phényl(alkyle en C₁-C₂) ou phényl(alcoxy en C₁-C₂) ;
R₉ et R₁₀ sont chacun indépendamment de l'autre un groupe alkyle en C₁-C₈ ; ou -C(H)=O- ;
X₄ et X₂ sont chacun indépendamment de l'autre un atome d'hydrogène ; un groupe alkyle en C₁-C₅ ; ou bien
X₁ et X₂, avec l'atome d'azote auquel ils sont liés, forment un noyau pyrrolidinyle ou pipéridinyle non-substitué ; et
X₃ est un atome d'hydrogène ou le groupe méthyle.

5. Mélange de formateurs de couleur selon l'une des revendications 1 à 4, contenant un formateur de couleur de formule (1) dans laquelle
R₂ est COR₈, et
R₈ est un atome d'hydrogène ; un groupe alkyle en C₁-C₄ ; alcoxy en C₁-C₄ ; phényle non-substitué ou substitué sur le noyau phényle par des substituants halogéno, alkyle en C₁-C₄, halogénalkyle en C₁-C₄ ou alcoxy en C₁-C₄ ; phényl(alkyle en C₁-C₂) ou phényl(alcoxy en C₁-C₂).

6. Mélange de formateurs de couleur selon l'une des revendications 1 à 5, caractérisé en ce qu'on utilise en tant que formateurs de couleur de formule (2) des composés dans lesquels :
R₁₀ est un groupe alkyle en C₁-C₈.

7. Mélange de formateurs de couleur selon l'une des revendications 1 à 6, caractérisé en ce qu'on utilise en tant que formateurs de couleur des composés de formule (3) dans laquelle :
X₄ et X₅ sont chacun indépendamment de l'autre un groupe phényle non-substitué ou substitué sur le noyau phényle par des substituants halogéno, nitro, alkyle en C₁-C₄, alcoxy en C₁-C₄, halogénalkyle en C₁-C₄ ou -NR₁₂R₁₃ ;
Y₃ est un groupe phényle non-substitué ou substitué sur le noyau phényle par des substituants halogéno, alkyle en C₁-C₄, halogénalkyle en C₁-C₄, alcoxy en C₁-C₄ ; et
le noyau benzène A est substitué par des substituants halogéno ; alkyle en C₁-C₅ ; alcoxy en C₁-C₅ ; ou NR₁₃R₁₄.

8. Mélange de formateurs de couleur selon l'une des revendications 1 à 7, contenant en tant que constituant (b) un composé de formule (4) dans laquelle
Y₁ et Y₂ sont chacun indépendamment de l'autre un groupe alkyle en C₃-C₅, et
R₉ est un groupe alkyle en C₁-C₅ ou -C(H)=O- ; et
R₁₁ est un atome d'hydrogène ou un groupe alkyle en C₁-C₅.

9. Mélange de formateurs de couleur selon la revendication 8, contenant un formateur de couleur (b) de formule (4) dans laquelle
Y₁ et Y₂ sont des groupes alkyle en C₄-C₅.

10. Mélange de formateurs de couleur selon la revendication 8 ou 9, caractérisé en ce que Y₁ et Y₂ ont les mêmes significations.

11. Mélange de formateurs de couleur selon la revendication 1, contenant en tant que constituant (a) un composé de formule (1) dans laquelle
R₁ est un atome d'hydrogène, un atome d'halogène ou le groupe méthyle ;
R₂ est COR₈ ;
n vaut 0, 1, 2, 3 ou 4 ;
R₃, quand n vaut 1, 2, 3 ou 4, est un atome d'halogène ; quand n vaut 1 ou 2, le groupe méthyle ; ou quand n vaut 1, un groupe nitro, amino, mono(alkyle en C₁-C₄)-amino ou di(alkyle en C₁-C₄)amino ;
R₈ est un atome d'hydrogène ; un groupe alkyle en C₁-C₄ ; alcoxy en C₁-C₄ ; phényle non-substitué ou substitué sur le noyau phényle par des substituants halogéno, alkyle en C₁-C₄, halogénalkyle en C₁-C₄ ou alcoxy en C₁-C₄ ; phényl(alkyle en C₁-C₂) ou phényl(alcoxy en C₁-C₂) ;
X₁ et X₂ sont chacun indépendamment de l'autre un atome d'hydrogène ; un groupe alkyle en C₁-C₅ ; ou bien
X₁ et X₂, avec l'atome d'azote auquel ils sont liés, forment un noyau pyrrolidinyle ou pipéridinyle non-substitué ; et
X₃ est un atome d'hydrogène ou le groupe méthyle ;
et/ou un composé de formule (2), dans laquelle
R₁₀ est un groupe alkyle en C₁-C₅ ;
et, en tant que constituant (b), un composé de formule (4), dans laquelle
Y₁ et Y₂ sont des groupes alkyle en C₃-C₅ et
R₉ est un groupe alkyle en C₁-C₅ ou -C(H)=O-, et
R₁₁ est un atome d'hydrogène ou un groupe alkyle en C₁-C₅.

12. Mélange de formateurs de couleur selon l'une des revendications 1 à 11, caractérisé en ce que le rapport en poids (a):(b) est de 1:5 à 1:1.

13. Mélange de formateurs de couleur selon l'une des revendications 1 à 12, caractérisé en ce que les constituants (a) et (b) se présentent à l'état cristallin.

14. Solution microencapsulé d'un mélange de formateurs de couleur, qui contient un composé de formule (1) et/ou de formule (2) et/ou de formule (3) et un composé de formule (4) ou un mélange de formateurs de couleur contenant au moins deux composés du constituant (b) selon la revendication 1.

15. Matériau d'enregistrement sensible à la pression, contenant un mélange de formateurs de couleur selon l'une des revendications 1 à 13.

16. Matériau d'enregistrement sensible à la chaleur, contenant un mélange de formateurs de couleur selon l'une des revendications 1 à 13.

17. Matériau d'enregistrement sensible à la chaleur selon la revendication 16, qui comporte une couche sensible à la chaleur, laquelle contient un formateur de couleur et un révélateur pour un formateur de couleur, caractérisé en ce que le formateur de couleur contient un mélange de formateurs de couleur selon l'une des revendications 1 à 11.

18. Composés de formule 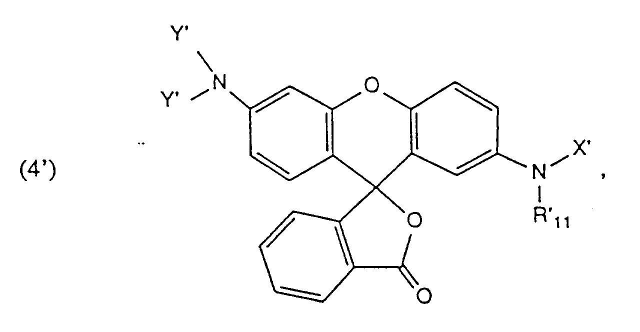 dans laquelle
X' est un groupe alkyle en C₁-C₅ ou -C(H)=O- ;
Y' est un groupe alkyle en C₄-C₅ ; et
R'₁₁ est un atome d'hydrogène ou un groupe alkyle en C₁-C₅.

19. Procédé pour préparer les composés de formule (4'), caractérisé en ce qu'on condense une benzophénone de formule 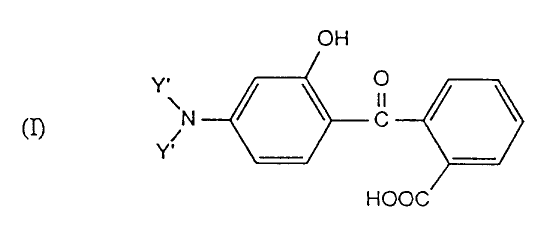 avec un phénol ou un phénoléther de formule 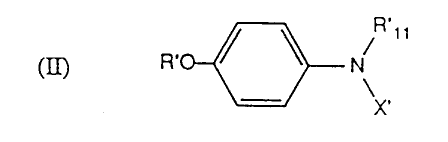 à des températures de préférence de 0 à 70°C dans de l'acide sulfurique à 50 à 100 %, éventuellement avec élimination d'un groupe formyl-protecteur, pour obtenir un phtalide de formule 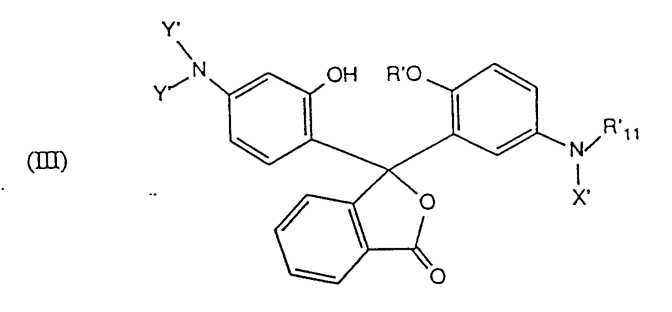 puis, à des températures de 20 à 100°C, on procède à une cyclisation pour obtenir un composé de formule (4'), où
X' et Y' et R'₁₁ ont les significations données dans la formule (4') et
R' est un atome d'hydrogène ou un groupe alkyle en C₁-C₄.
